# EUROPEAN PATENT APPLICATION

(11) **EP 4 494 483 A1**
(43) Date of publication of application: **22.01.2025**
(21) Application number: 23770903.5
(22) Date of filing: 17.03.2023
(51) Int. Cl.: A23L 33/125, A23L 33/10, A23L 33/135, A61K 31/7004, A61K 31/7008, A61K 31/7012, A61K 31/7016, A61K 31/702, A61K 35/20, A61P 1/00, A61P 1/04, A61P 1/12, A61P 1/14, A61P 1/16, A61P 3/00, A61P 9/06, A61P 11/06, A61P 13/12, A61P 17/00, A61P 25/00

(54) **COMPOSITION FOR CONTROLLING PROLIFERATION OF BACTERIUM IN INTESTINE, AND USE THEREOF**

(30) Priority: 18.03.2022 JP 2022044397; 18.03.2022 JP 2022044398; 18.03.2022 JP 2022044399; 18.03.2022 JP 2022044400; 18.03.2022 JP 2022044401; 18.03.2022 JP 2022044402; 18.03.2022 JP 2022044403; 18.03.2022 JP 2022044404; 18.03.2022 JP 2022044405; 18.03.2022 JP 2022044406; 18.03.2022 JP 2022044407
(71) Applicant: MEIJI CO., LTD, Chuo-ku Tokyo 104-8306 (JP)
(72) Inventor: FUJIWARA Shin, Hachioji-shi, Tokyo 192-0919 (JP); TAKEDA Mariko, Hachioji-shi, Tokyo 192-0919 (JP)
(74) Representative: TBK
(86) International application number: PCT/JP2023/010496
(87) International publication number: WO 2023/176950

(57) **Abstract**

A means for controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal tract is provided. An object of the present invention is to provide a novel material that can control growth of bacteria of the genus *Faecalibacterium.* There is provided a composition for controlling growth of target bacteria in intestinal bacterial microbiota, which contains any selected from the group consisting of a human milk oligosaccharide and a constituent sugar of a human milk oligosaccharide.

## Description

### Technical Field

The present invention relates to a composition for controlling growth of bacteria in the intestinal tract, more specifically, any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella,* and use thereof.

### Background Art

Bacteria of the genus *Faecalibacterium* are predominant intestinal bacteria that account for about 5% of the total intestinal bacteria in healthy adults, and recently attract attention as bacterial species having favorable physiological functions for human health, such as butyrate production and anti-inflammatory activity. With regard to bacteria of the genus *Faecalibacterium,* Patent document 1 describes use of riboflavin, riboflavin phosphate, or a physiologically acceptable salt thereof for production of a food composition, pharmaceutical composition, food, or dietary supplement for selectively stimulating *Faecalibacterium prausnitzii* in the gastrointestinal tract of animals.

Bacteria of the genus *Akkermansia,* especially *A. muciniphila,* are bacteria that inhabit the intestines of mammals including humans. Patent document 2 describes an agent for increasing number of bacteria of the genus *Akkermansia* and/or *Bacteroides,* comprising a novel mucin-type glycoprotein containing sulfate group and sialic acid obtained from rays as an active ingredient.

Bacteria of the genus *Blautia* are predominant intestinal bacteria, accounting for about 5% of the total intestinal bacteria in healthy adults. With respect to bacteria of the genus *Blautia,* Patent document 3 describes a solid composition for oral administration for treating non-human mammals suffering from gastrointestinal diseases and so forth, containing multiple kinds of microorganisms of the genus *Blautia* and others. In addition, Patent document 4 describes a method for treating an immune disorder in a subject, comprising administering a bacterial composition containing strain A of the genus *Blautia.*

Bacteria of the genus *Subdoligranulum* are bacteria that inhabit in the intestines of mammals including humans. With respect to bacteria of the genus *Subdoligranulum,* Patent document 5 describes a composition of a purified strain of bacteria of the genus *Subdoligranulum* and other bacteria, and a method for treating pathogen infection such as *Clostridium difficile* infection by administering the composition to a subject having the pathogen infection. In addition, Patent document 6 describes use of grape skins or a composition containing them as prebiotics to increase the intestinal levels and/or activity of butyrate-producing bacteria, such as *Faecalibacterium prausnitzii,* phylogroup II (PHGI) of *F. prausnitzii,* phyletic group II (PHGII) of *F. prausnitzii, Roseburia hominis,* and *Subdoligranulum variabile,* in a subj ect.

Bacteria of the genus *Bilophila* constitute one class of hydrogen sulfide-producing bacteria in the intestinal tract. With respect to bacteria of the genus *Bilophila,* Patent document 7 describes an anti-gram-negative bacteria pharmaceutical composition containing a therapeutically effective amount of robenidine or a therapeutically acceptable salt thereof, and a compound or active material that removes or reduces cell wall integrity in gram-negative bacteria, and mentions *Bilophila wadsworthia* as one of the gram-negative bacteria. In addition, Patent document 8 describes an antibacterial agent for non-spore anaerobic gram-negative rods, containing a sasa extract, and mentions bacteria of the genus *Bilophila* as one class of the non-spore anaerobic gram-negative rods. Furthermore, Patent document 9 describes a *Bilophila wadsworthia* growth inhibitor used for prevention and/or treatment of diseases caused by *Bilophila wadsworthia,* which contains *Bifidobacterium longum* ATCC BAA-999 as the active ingredient. *Bilophila wadsworthia* proliferates with a meat-based diet.

Bacteria of the genus *Sutterella* constitute one class of bacteria in the human intestine and have been reported to be associated with a variety of diseases. With respect to bacteria of the genus *Sutterella,* it has been reported that when kestose was daily administered to healthy adult dogs with their normal diet for 8 weeks, there was observed a tendency that *Bifidobacterium* bacteria in their feces increase, while bacteria of the genera *Sutterella* and *Bacteroides* decrease (Non-patent document 1). In addition, it has been reported that *Gelidium pacificum Okamura* polysaccharides (sulfated polysaccharides, GPOP-1) significantly increased the relative amounts of *Bacteroides, Oscillospira,* and *Bifidobacterium* bacteria, and decreased the relative amounts of *Parabacteroides, Sutterella,* and AF 12 bacteria in the intestinal microbiota of mice with antibiotic-associated diarrhea (AAD) (Non-Patent document 2). Furthermore, Patent document 10 describes a bacterial count-suppressing agent for *Fusobacterium* bacteria and/or *Sutterella* bacteria containing 1-kestose as an active ingredient, and describes that this agent can be used for prevention or treatment of acute hemorrhagic diarrhea.

By the way, human milk oligosaccharides (HMOs) are the third most abundant solid component in breast milk after lactose and lipids. It is known that feeding 2'-fucosyllactose, 3-fucosyllactose, and lactodifucotetraose, types of human milk oligosaccharides, causes bifidobacteria to grow (Non-patent document 3). It has also been reported that 2'-fucosyllactose affected *Parabacteroides* bacteria in the intestine when administered to mice after ileal resection (Non-patent document 4).

With respect to HMOs, Patent document 11 describes a method for reducing or decreasing the risk of recurrence of inflammatory bowel disease (IBD), comprising administering an effective amount of a 2'-fucosyllactose (2'-FL) compound to a subject in need thereof, wherein the subject is a human IBD patient who has received or is receiving an anti-inflammatory therapy. This reference describes that the treatment of IBD with an anti-inflammatory therapy (e.g., use of anti-TNF inhibitor) suppresses intestinal inflammation and promotes cellular responsiveness of intestinal endothelial cells to butyrate, but does not increase the abundance of beneficial intestinal bacteria, while the combination therapy with 2'-FL and anti-TNF provides direct modulation of the beneficial microbiota and can increase microbial butyrate production while promoting butyrate responsiveness of cells to anti-TNF therapy, thereby enhancing sustained clinical remission of IBD. Patent document 12 describes a synthetic composition characterized by containing an effective amount of human milk monosaccharide (HMS) or HMO or both, which is for treatment of patients with irritable bowel syndrome (IBS) with one or more of abnormal bacterial growth, dysbiosis and mucosal barrier damage; reduction of IBS symptoms; relief of chronic symptoms of abdominal pain, abdominal discomfort and/or bloating, chronic symptoms of changes in bowel movement patterns, such as soft stool or frequent bowel movements, diarrhea and constipation; prevention of recurrence of IBS in former patients; increase of *Bifidobacterium* bacteria, preferably *Bifidobacterium adrescentis,* in the colon of patients with IBS; and prevention of IBS or IBS symptoms in patients who are or have been treated with antibiotics. Further, Non-patent document 5 reported that, as an effect of feeding 2'-FL-containing nutritional supplement in adults with IBS or ulcerative colitis, the number of *Bifidobacterium* bacteria and *Faecalibacterium prausnitzii* in stool increased after 6 weeks of the intervention. Non-patent document 6 reported that administration of 3'- or 6'-sialyllactose provided observation of difference in bacterial microbiota in the proximal and distal colon of neonatal pigs in contrast to a control group, which was caused by increase of *Collinsella aerofaciens* (phylum *Actinobacteria*), *Ruminococcus* and *Faecalibacterium* bacteria (phylum *Firmicutes*), and *Prevotella* bacteria (phylum *Bacteroides*)*.*

Also with respect to HMOs, Patent document 13 describes a synthetic composition for use in increasing the abundance of bacteria of the genus *Akkermansia* present in the human gastrointestinal tract, which contains at least one kind of HMO. This reference describes experimental results that everyday ingestion of 2'-fucosylated lactose, lacto-N-neotetraose, or a mixture thereof by healthy adults aged 18 to 60 years provided an increase in the amount of *Akkermansia* bacteria in the feces after 2 weeks and an increase in the amount of *Akkermansia* bacteria in the distal part of the colon region during the 3-week study period. Non-patent document 7 also reported that A. *muciniphila* can grow in human milk and can degrade HMOs. Non-patent document 8 examined four strains of the genus *Akkermansia* and reported that they differ in their ability to degrade HMOs.

Furthermore, with respect to HMOs, Non-patent document 9 describes that when male pigs were fed diets containing bovine milk oligosaccharides (BMOS), 2'-fucosyllactose and lacto-N-neotetraose (HMO), or both (BMOS + HMO), the microbial composition of the ascending colon was not affected in the HMO group, but the relative proportion of certain taxonomic groups including *Blautia* bacteria increased in that group compared with the other groups.

### Prior Art References

### Patent documents

Patent document 1: Japanese Patent Publication (Kokai) No. 2015-535280
Patent document 2: WO2019/194130
Patent document 3: WO2018/218211 (Japanese Patent Publication (Kohyo) No. 2020-521813)
Patent document 4: WO2019/099482 (Japanese Patent Publication (Kohyo) No. 2021-502964)
Patent document 5: WO2017/218680 (Japanese Patent Publication (Kohyo) No. 2019-517828)
Patent document 6: WO2018/220237 (Japanese Patent Publication (Kohyo) No. 2020-521801)
Patent document 7: WO2014/176634 (Japanese Patent Publication (Kohyo) No. 2016-520044, Japanese Patent No. 6254257)
Patent document 8: Japanese Patent Publication (Kokai) No. 2010-209066
Patent document 9: Japanese Patent Publication (Kokai) No. 2016-69309 (Japanese Patent No. 6247185)
Patent document 10: WO2019/195942 (Japanese Patent Publication (Kohyo) No. 2021-521203)
Patent document 11: Japanese Patent Publication (Kokai) No. 2020-513014
Patent document 12: Japanese Patent Publication (Kokai) No. 2021-152062
Patent document 13: WO2019/106620 (Japanese Patent Publication (Kohyo) No. 2021-504420)

### Non-patent documents

Non-patent document 1: Ide K, Shinohara M, Yamagishi S, Endo A, Nishifuji K, Tochio T. Kestose supplementation exerts bifidogenic effect within fecal microbiota and increases fecal butyrate concentration in dogs. J Vet Med Sci. 2020;82(1):1-8. doi:10.1292/jvms. 19-0071
Non-patent document 2: Cui M, Zhou R, Wang Y, Zhang M, Liu K, Ma C. Beneficial effects of sulfated polysaccharides from the red seaweed Gelidium pacificum Okamura on mice with antibiotic-associated diarrhea. Food Funct. 2020;11(5):4625-4637. doi:10.1039/d0fo00598c
Non-patent document 3: Yu ZT, Chen C, Newburg DS. Utilization of major fucosylated and sialylated human milk oligosaccharides by isolated human gut microbes. Glycobiology. 2013;23(11):1281-1292. doi:10.1093/glycob/cwt065
Non-patent document 4: The human milk oligosaccharide 2'-fucosyllactose augments the adaptive response to extensive intestinal. Am J Physiol Gastrointest Liver Physiol. 2016 Mar 15; 310(6): G427-G438
Non-patent document 5: Impact of 2'-Fucosyllactose on Gut Microbiota Composition in Adults with Chronic Gastrointestinal Conditions: Batch Culture Fermentation Model and Pilot Clinical Trial Findings. Nutrients. 2021;13(3):938
Non-patent document 6: Dietary Isomers of Sialyllactose Increase Ganglioside Sialic Acid Concentrations in the Corpus Callosum and Cerebellum and Modulate the Colonic Microbiota of Formula-Fed Piglets. J Nutr. 2016;146(2):200-208
Non-patent document 7: Kostopoulos I, Elzinga J, Ottman N, et al. Akkermansia muciniphila uses human milk oligosaccharides to thrive in the early life conditions in vitro. Sci Rep. 2020;10(1): 14330. Published 2020 Aug 31. doi:10.1038/s41598-020-71113-8
Non-patent document 8: Luna E, Parkar SG, Kirmiz N, et al. Utilization efficiency of human milk oligosaccharides by human-associated Akkermansia is strain-dependent [published online ahead of print, 2021 Oct 20]. Appl Environ Microbiol. 2021;AEM0148721. doi:10.1128/AEM.01487-21
Non-patent document 9: Wang M, Monaco MH, Hauser J, Yan J, Dilger RN, Donovan SM. Bovine Milk Oligosaccharides and Human Milk Oligosaccharides Modulate the Gut Microbiota Composition and Volatile Fatty Acid Concentrations in a Preclinical Neonatal Model. Microorganisms. 2021;9(5):884. Published 2021 Apr 21. doi:10.3390/microorganisms9050884

### Summary of the Invention

### Object to be achieved by the invention

By controlling growth of specific bacteria in the intestinal tract, various diseases associated with the control of growth of the bacteria may be prevented or ameliorated.

Currently, only a few materials are known for control of bacteria in the intestinal tract. It would be desirable to have a new material that can control growth of any bacteria in the intestinal tract selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella.*

### Means for achieving the object

The present invention provides the followings.
[1] A composition for controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, which contains any selected from the group consisting of a human milk oligosaccharide containing sialic acid as a constituent sugar and a constituent sugar thereof.
[2] The composition according to 1, which contains any selected from the group consisting of N-acetylneuraminic acid, 3'-sialyllactose, and 6'-sialyllactose.
[3] The composition according to 1 or 2, which contains any selected from the group consisting of N-acetylneuraminic acid and 3'-sialyllactose.
[4] The composition according to 2, which is for controlling growth of any bacteria selected from the group consisting of bacteria of the genus *Faecalibacterium,* bacteria of the genus *Akkermansia,* bacteria of the genus *Blautia,* and bacteria of the genus *Subdoligranulum.*
[5] The composition according to 1, which contains any selected from the group consisting of N-acetylneuraminic acid, 3'-sialyllactose, 6'-sialyllactose, fucose, and lactose, and is for controlling growth of any bacteria selected from the group consisting of bacteria of the genus *Faecalibacterium* and bacteria of the genus *Akkermansia.*
[6] The composition according to 1, which contains lactose, and is for controlling growth of any bacteria selected from the group consisting of bacteria of the genus *Blautia* and bacteria of the genus *Sutterella.*
[7] The composition according to any one of 1 to 6, which is for use as a prebiotic or synbiotic.
[8] A composition for treating a disease or condition that can be ameliorated by controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, which contains any selected from the group consisting of a human milk oligosaccharide containing sialic acid as a constituent sugar and a constituent sugar thereof.
[9] The composition according to 8, which contains any selected from the group consisting of N-acetylneuraminic acid, 3'-sialyllactose, and 6'-sialyllactose.
[10] The composition according to any one of 1 to 7, which is for promoting growth of any bacteria selected from the group consisting of bacteria of the genus *Faecalibacterium,* bacteria of the genus *Akkermansia,* bacteria of the genus *Blautia,* and bacteria of the genus *Subdoligranulum,* or for suppressing growth of any bacteria selected from the group consisting of bacteria of the genus *Bilophila* and bacteria of the genus *Suterella.*
[11] The composition according to 8 or 9, which is for treating a disease or condition that can be ameliorated by promoting growth of any bacteria selected from the group consisting of bacteria of the genus *Faecalibacterium,* bacteria of the genus *Akkermansia,* bacteria of the genus *Blautia,* and bacteria of the genus *Subdoligranulum,* or for treating a disease or condition that can be ameliorated by suppressing growth of any bacteria selected from the group consisting of bacteria of the genus *Bilophila* and bacteria of the genus *Sutterella.*
[12] A composition for use in a method for controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, which contains any selected from the group consisting of a human milk oligosaccharide containing sialic acid as a constituent sugar and a constituent sugar thereof, use of any selected from the group consisting of a human milk oligosaccharide containing sialic acid as a constituent sugar and a constituent sugar thereof in manufacture of a composition for controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, a method or non-therapeutic method for controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, which comprises the step of administering a composition containing any selected from the group consisting of a human milk oligosaccharide containing sialic acid as a constituent sugar and a constituent sugar thereof to a subject, or use or non-therapeutic use of a composition containing any selected from the group consisting of a human milk oligosaccharide containing sialic acid as a constituent sugar and a constituent sugar thereof for controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota.
   The present invention provides the followings.
[13] The composition, use in manufacture, method or non-therapeutic method, or use or non-therapeutic use according to 12, wherein the human milk oligosaccharide containing sialic acid as a constituent sugar and the constituent sugar thereof are any selected from the group consisting of N-acetylneuraminic acid, 3'-sialyllactose, and 6'-sialyllactose.
[14] The composition, use in manufacture, method or non-therapeutic method, or use or non-therapeutic use according to 12 or 13, wherein the human milk oligosaccharide containing sialic acid as a constituent sugar and the constituent sugar thereof are any selected from the group consisting of N-acetylneuraminic acid and 3'-sialyllactose.
[15] The composition, use in manufacture, method or non-therapeutic method, or use or non-therapeutic use according to 13, wherein the control of growth is control of growth of any bacteria selected from the group consisting of bacteria of the genus *Faecalibacterium,* bacteria of the genus *Akkermansia,* bacteria of the genus *Blautia,* and bacteria of the genus *Subdoligranulum.*
[16] The composition, use in manufacture, method or non-therapeutic method, or use or non-therapeutic use according to 12, wherein the human milk oligosaccharide containing sialic acid as a constituent sugar and the constituent sugar thereof are any selected from the group consisting of N-acetylneuraminic acid, 3'-sialyllactose, 6'-sialyllactose, fucose and lactose, and the control of growth is control of growth of any bacteria selected from the group consisting of bacteria of the genus *Faecalibacterium* and bacteria of the genus *Akkermansia.*
[17] The composition, use in manufacture, method or non-therapeutic method, or use or non-therapeutic use according to 12, wherein the constituent sugar of the human milk oligosaccharide containing sialic acid as a constituent sugar is lactose, and the control of growth is control of growth of any bacteria selected from the group consisting of bacteria of the genus *Blautia* and bacteria of the genus *Sutterella.*
[18] The use in manufacture, method or non-therapeutic method, or use or non-therapeutic use according to any one of 12 to 17, wherein the composition is for use as a prebiotic or synbiotic.
[19] A composition for use in a method for treating a disease or condition that can be ameliorated by controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, which contains any selected from the group consisting of a human milk oligosaccharide containing sialic acid as a constituent sugar and a constituent sugar thereof, use of any selected from the group consisting of a human milk oligosaccharide containing sialic acid as a constituent sugar and a constituent sugar thereof in manufacture of a composition for treating a disease or condition that can be ameliorated by controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, a method or non-therapeutic method for treating a disease or condition that can be ameliorated by controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, which comprises the step of administering a composition containing any selected from the group consisting of a human milk oligosaccharide containing sialic acid as a constituent sugar and a constituent sugar thereof to a subject, or use or non-therapeutic use of a composition containing any selected from the group consisting of a human milk oligosaccharide containing sialic acid as a constituent sugar and a constituent sugar thereof for treating a disease or condition that can be ameliorated by controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota.
[20] The composition, use in manufacture, method or non-therapeutic method, or use or non-therapeutic use according to 19, wherein the human milk oligosaccharide containing sialic acid as a constituent sugar and constituent sugar thereof are any selected from the group consisting of N-acetylneuraminic acid, 3'-sialyllactose, and 6'-sialyllactose.
[21] The composition, use in manufacture, method or non-therapeutic method, or use or non-therapeutic use according to any one of 12 to 18, wherein the control of growth is promotion of growth of any bacteria selected from the group consisting bacteria of the genus *Faecalibacterium,* bacteria of the genus *Akkermansia,* bacteria of the genus *Blautia,* and bacteria of the genus *Subdoligranulum,* or suppression of growth of any bacteria selected from the group consisting of bacteria of the genus *Bilophila* and bacteria of the genus *Suterella.*
[22] The composition, use in manufacture, method or non-therapeutic method, or use or non-therapeutic use according to 19 or 20, wherein the control of growth is promotion of growth of any bacteria selected from the group consisting bacteria of the genus *Faecalibacterium,* bacteria of the genus *Akkermansia,* bacteria of the genus *Blautia,* and bacteria of the genus *Subdoligranulum,* or suppression of growth of any bacteria selected from the group consisting of bacteria of the genus *Bilophila* and bacteria of the genus *Suterella.*

The present invention provides the followings.
[1] A composition for controlling growth of bacteria of the genus *Faecalibacterium* in the intestinal bacterial microbiota, which contains any selected from the group consisting of a human milk oligosaccharide and a constituent sugar of a human milk oligosaccharide.
[2] The composition according to 1, which contains any selected from the group consisting of a fucosylated human milk oligosaccharide, a sialylated human milk oligosaccharide, a human milk oligosaccharide containing lactose as a constituent sugar, a human milk oligosaccharide containing fucose as a constituent sugar, a human milk oligosaccharide containing sialic acid as a constituent sugar, lactose, sialic acid, and fucose.
[3] The composition according to 1 or 2, which contains any selected from the group consisting of 2'-fucosyllactose, 3'-sialyllactose, 6'-sialyllactose, lactose, N-acetylneuraminic acid, and fucose.
[4] The composition according to 1 or 2, which contains any selected from the group consisting of 3'-sialyllactose, 6'-sialyllactose, lactose, N-acetylneuraminic acid, and fucose.
[5] The composition according to any one of 1 to 4, which is for use as a prebiotic or synbiotic.
[6] The composition according to any one of 1 to 5, which is for use in treating any selected from the group consisting of irritable bowel syndrome, inflammatory bowel disease, Parkinson's disease, cancer, cognitive decline, and atopic dermatitis.
[7] A composition for treating a disease or condition that can be ameliorated by controlling bacteria of the genus *Faecalibacterium* in the intestinal tract, which contains any selected from the group consisting of a human milk oligosaccharide and a constituent sugar of a human milk oligosaccharide.
[8] A composition for treating any selected from the group consisting of irritable bowel syndrome, inflammatory bowel disease, Parkinson's disease, cancer, cognitive decline, and atopic dermatitis, which contains any selected from the group consisting of a human milk oligosaccharide and a constituent sugar of a human milk oligosaccharide.
[9] An anti-inflammatory composition, which contains any selected from the group consisting of a human milk oligosaccharide and a constituent sugar of a human milk oligosaccharide.
[10] The composition according to any one of 7 to 9, which contains any selected from the group consisting of 3'-sialyllactose, 6'-sialyllactose, lactose, N-acetylneuraminic acid, and fucose.
[11] The composition according to any one of 1 to 7, wherein the control of growth is promotion of growth.
[12] A composition for use in a method for controlling growth of bacteria of the genus *Faecalibacterium* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of bacteria of the genus *Faecalibacterium* in the intestinal bacterial microbiota, a method for controlling growth of bacteria of the genus *Faecalibacterium* in the intestinal bacterial microbiota, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for controlling growth of bacteria of the genus *Faecalibacterium* in the intestinal bacterial microbiota.
[13] The composition according to in 12, which contains any selected from the group consisting of a fucosylated human milk oligosaccharide, a sialylated human milk oligosaccharide, a human milk oligosaccharide containing lactose as a constituent sugar, a human milk oligosaccharide containing fucose as a constituent sugar, a human milk oligosaccharide containing sialic acid as a constituent sugar, lactose, sialic acid, and fucose; or the use in manufacture, method, or use according to 12, wherein the composition is such a composition.
[14] The composition according to 12 or 13, which contains any selected from the group consisting of 2'-fucosyllactose, 3'-sialyllactose, 6'-sialyllactose, lactose, N -acetylneuraminic acid, and fucose; or the use in manufacture, method, or use according to 12 or 13, wherein the composition is such a composition.
[15] The composition according to 12 or 13, which contains any selected from the group consisting of 3'-sialyllactose, 6'-sialyllactose, lactose, N-acetylneuraminic acid, and fucose; or the use in manufacture, method, or use according to 12 or 13, wherein the composition is such a composition.
[16] The composition according to any one of 12 to 15, which is for use as a prebiotic or synbiotic; the use in manufacture according to any one of 12 to 15, wherein the composition is such a composition; the method according to any one of 12 to 15, which comprises the step of administering the composition as a prebiotic or synbiotic; or the use of a composition according to any one of 12 to 15, wherein the composition is used as a prebiotic or synbiotic.
[17] The composition according to any one of 12 to 16, which is for use in treating any selected from the group consisting of irritable bowel syndrome, inflammatory bowel disease, Parkinson's disease, cancer, cognitive decline, and atopic dermatitis; the use in manufacture according to any one of 12 to 16, wherein the composition is such a composition; the method according to any one of 12 to 16, which is for treating any selected from the group consisting of irritable bowel syndrome, inflammatory bowel disease, Parkinson's disease, cancer, cognitive decline, and atopic dermatitis; or the use of a composition according to any one of 12 to 16, which is for such a treatment.
[18] A composition for use in a method for treating a disease or condition that can be ameliorated by controlling growth of bacteria of the genus *Faecalibacterium* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Faecalibacterium* in the intestinal tract, a method for treating a disease or condition that can be ameliorated by controlling growth of bacteria of the genus *Faecalibacterium* in the intestinal tract, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating a disease or condition that can be ameliorated by controlling growth of bacteria of the genus *Faecalibacterium* in the intestinal tract.
[19] A composition for use in a method for treating any selected from the group consisting of irritable bowel syndrome, inflammatory bowel disease, Parkinson's disease, cancer, cognitive decline, and atopic dermatitis, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating any selected from the group consisting of irritable bowel syndrome, inflammatory bowel disease, Parkinson's disease, cancer, cognitive decline, and atopic dermatitis, a method for treating any selected from the group consisting of irritable bowel syndrome, inflammatory bowel disease, Parkinson's disease, cancer, cognitive decline, and atopic dermatitis, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating any selected from the group consisting of irritable bowel syndrome, inflammatory bowel disease, Parkinson's disease, cancer, cognitive decline, and atopic dermatitis.
[20] A composition for use in an anti-inflammatory method, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of an anti-inflammatory composition, an anti-inflammatory method comprising the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for anti-inflammation.
[21] The composition according to any one of 18 to 20, which contains any selected from the group consisting of 3'-sialyllactose, 6'-sialyllactose, lactose, N-acetylneuraminic acid, and fucose; or the use in manufacture, method, or use according to any one of 18 to 20, wherein the composition is such a composition.
[22] The composition, use in manufacture of a composition, method, or use according to any one of 12 to 18, wherein the control of growth is promotion of growth.

The present invention provides the followings.
[1] A composition for controlling growth of bacteria of the genus *Akkermansia* in the intestinal bacterial microbiota, which contains any selected from the group consisting of a sialylated human milk oligosaccharide, a human milk oligosaccharide containing sialic acid as a constituent sugar, lactose, sialic acid, and fucose.
[2] The composition according to 1, which contains a human milk oligosaccharide containing N-acetylneuraminic acid as a constituent sugar.
[3] The composition according to 2, which contains any selected from the group consisting of 3'-sialyllactose, 6'-sialyllactose, lactose, N-acetylneuraminic acid, and fucose.
[4] The composition according to 3, which contains any selected from the group consisting of lactose, N-acetylneuraminic acid, and fucose.
[5] The composition according to any one of 1 to 4, which is for use as a prebiotic or synbiotic.
[6] The composition according to any one of 1 to 5, which is for use in treating any selected from the group consisting of obesity, abnormal sugar metabolism, inflammatory bowel disease, autism, and atopic dermatitis.
[7] A composition for treating a disease or condition that can be ameliorated by controlling growth of bacteria of the genus *Akkermansia* in the intestinal tract, which contains any selected from the group consisting of a human milk oligosaccharide and a constituent sugar of a human milk oligosaccharide.
[8] A composition for treating any selected from the group consisting of obesity, abnormal sugar metabolism, inflammatory bowel disease, autism, and atopic dermatitis, which contains any selected from the group consisting of a human milk oligosaccharide and a constituent sugar of a human milk oligosaccharide.
[9] The composition according to 7 or 8, which contains any selected from the group consisting of a sialylated human milk oligosaccharide, a human milk oligosaccharide containing sialic acid as a constituent sugar, lactose, sialic acid, and fucose.
[10] The composition according to any one of 1 to 7, wherein the control of growth is promotion of growth.
[11] A composition for use in a method for controlling growth of bacteria of the genus *Akkermansia* in the intestinal bacterial microbiota, which contains any selected from the group consisting of a sialylated human milk oligosaccharide, a human milk oligosaccharide containing sialic acid as a constituent sugar, lactose, sialic acid, and fucose, use of any selected from the group consisting of a sialylated human milk oligosaccharide, a human milk oligosaccharide containing sialic acid as a constituent sugar, lactose, sialic acid, and fucose in manufacture of a composition for controlling growth of bacteria of the genus *Akkermansia* in the intestinal bacterial microbiota, a method for controlling growth of bacteria of the genus *Akkermansia* in the intestinal bacterial microbiota, which comprises the step of administering a composition containing any selected from the group consisting of a sialylated human milk oligosaccharide, a human milk oligosaccharide containing sialic acid as a constituent sugar, lactose, sialic acid, and fucose to a subject, or use of a composition containing any selected from the group consisting of a sialylated human milk oligosaccharide, a human milk oligosaccharide containing sialic acid as a constituent sugar, lactose, sialic acid, and fucose for controlling growth of bacteria of the genus *Akkermansia* in the intestinal bacterial microbiota.
[12] The composition according to 11, which contains a human milk oligosaccharide containing N-acetylneuraminic acid as a constituent sugar; or the use in manufacture, method, or use according to 11, wherein the composition is such a composition.
[13] The composition according to 11 or 12, which contains any selected from the group consisting of 3'-sialyllactose, 6'-sialyllactose, lactose, N-acetylneuraminic acid, and fucose, or the use in manufacture, method, or use according to 11 or 12, wherein the composition is such a composition.
[14] The composition according to 11 or 13, which contains any selected from the group consisting of lactose, N-acetylneuraminic acid, and fucose; or the use in manufacture, method, or use according to 11 or 13, wherein the composition is such a composition.
[15] The composition according to any one of 11 to 14, which is for use as a prebiotic or synbiotic; the use in manufacture according to any one of 11 to 14, wherein the composition is such a composition; the method according to any one of 11 to 14, which comprises the step of administering the composition as a prebiotic or synbiotic; or the use of a composition according to any one of 11 to 14, wherein the composition is used as a prebiotic or synbiotic.
[16] The composition according to any one of 11 to 15, which is for use in treating any selected from the group consisting of obesity, abnormal sugar metabolism, inflammatory bowel disease, autism, and atopic dermatitis; the use in manufacture according to any one of 11 to 15, wherein the composition is such a composition; the method according to any one of 11 to 15, which comprises the step of administering the composition for treating any selected from the group consisting of obesity, abnormal sugar metabolism, inflammatory bowel disease, autism, and atopic dermatitis; or the use of a composition according to any one of 11 to 15, which is for use in treating any selected from the group consisting of obesity, abnormal sugar metabolism, inflammatory bowel disease, autism, and atopic dermatitis.
[17] A composition for treating a disease or condition that can be ameliorated by controlling growth of bacteria of the genus *Akkermansia* in the intestinal tract, which contains any selected from the group consisting of a sialylated human milk oligosaccharide, a human milk oligosaccharide containing sialic acid as a constituent sugar, lactose, sialic acid, and fucose, use of any selected from the group consisting of a sialylated human milk oligosaccharide, a human milk oligosaccharide containing sialic acid as a constituent sugar, lactose, sialic acid, and fucose in manufacture of a composition for treating a disease or condition that can be ameliorated by controlling growth of bacteria of the genus *Akkermansia* in the intestinal tract, a method for treating a disease or condition that can be ameliorated by controlling growth of bacteria of the genus *Akkermansia* in the intestinal tract, which comprises the step of administering a composition containing any selected from the group consisting of a sialylated human milk oligosaccharide, a human milk oligosaccharide containing sialic acid as a constituent sugar, lactose, sialic acid, and fucose to a subject, or use of a composition containing any selected from the group consisting of a sialylated human milk oligosaccharide, a human milk oligosaccharide containing sialic acid as a constituent sugar, lactose, sialic acid, and fucose for treating a disease or condition that can be ameliorated by controlling growth of bacteria of the genus *Akkermansia* in the intestinal tract.
[18] A composition for treating any selected from the group consisting of obesity, abnormal sugar metabolism, inflammatory bowel disease, autism, and atopic dermatitis, which contains any selected from the group consisting of a sialylated human milk oligosaccharide, a human milk oligosaccharide containing sialic acid as a constituent sugar, lactose, sialic acid, and fucose, use of any selected from the group consisting of a sialylated human milk oligosaccharide, a human milk oligosaccharide containing sialic acid as a constituent sugar, lactose, sialic acid, and fucose in manufacture of a composition for treating any selected from the group consisting of obesity, abnormal sugar metabolism, inflammatory bowel disease, autism, and atopic dermatitis, a method for treating any selected from the group consisting of obesity, abnormal sugar metabolism, inflammatory bowel disease, autism, and atopic dermatitis, which comprises the step of administering a composition containing any selected from the group consisting of a sialylated human milk oligosaccharide, a human milk oligosaccharide containing sialic acid as a constituent sugar, lactose, sialic acid, and fucose to a subject, or use of a composition containing any selected from the group consisting of a sialylated human milk oligosaccharide, a human milk oligosaccharide containing sialic acid as a constituent sugar, lactose, sialic acid, and fucose for treating any selected from the group consisting of obesity, abnormal sugar metabolism, inflammatory bowel disease, autism, and atopic dermatitis.
[19] The composition according to 17 or 18, which contains any selected from the group consisting of a sialylated human milk oligosaccharide, a human milk oligosaccharide containing sialic acid as a constituent sugar, lactose, sialic acid, and fucose; or the use in manufacture, method, or use according to 17 or 18, wherein the composition is such a composition.
[20] The composition, use of a composition in manufacture, method, or use according to any one of 11 to 17, wherein the control of growth is promotion of growth.

The present invention provides the followings
[1] A composition for controlling growth of bacteria of the genus *Blautia* in the intestinal bacterial microbiota, which contains any selected from the group consisting of a human milk oligosaccharide and a constituent sugar of a human milk oligosaccharide.
[2] The composition according to 1, which contains any selected from the group consisting of a fucosylated human milk oligosaccharide, a sialylated human milk oligosaccharide, a human milk oligosaccharide containing lactose as a constituent sugar, a human milk oligosaccharide containing fucose as a constituent sugar, a human milk oligosaccharide containing sialic acid as a constituent sugar, lactose, sialic acid, and fucose.
[3] The composition according to 1 or 2, which contains any selected from the group consisting of 2'-fucosyllactose, 3'-sialyllactose, 6'-sialyllactose, lactose, N-acetylneuraminic acid, and fucose.
[4] The composition according to any one of 1 to 3, which is for use as a prebiotic or synbiotic.
[5] The composition according to any one of 1 to 4, which is for use in treating any selected from the group consisting of obesity, abnormal sugar metabolism, diabetes mellitus, cancer, and inflammatory bowel disease.
[6] A composition for treating a disease or condition that can be ameliorated by controlling growth of bacteria of the genus *Blautia* in the intestinal tract, which contains any selected from the group consisting of a human milk oligosaccharide and a constituent sugar of a human milk oligosaccharide.
[7] A composition for treating any selected from the group consisting of obesity, abnormal sugar metabolism, diabetes mellitus, cancer, and inflammatory bowel disease, which contains any selected from the group consisting of a human milk oligosaccharide and a constituent sugar of a human milk oligosaccharide.
[8] The composition according to any one of 1 to 6, wherein the control of growth is promotion of growth.
[9] A composition for use in a method for controlling growth of bacteria of the genus *Blautia* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of bacteria of the genus *Blautia* in the intestinal bacterial microbiota, a method for controlling growth of bacteria of the genus *Blautia* in the intestinal bacterial microbiota, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for controlling growth of bacteria of the genus *Blautia* in the intestinal bacterial microbiota.
[10] The composition according to 9, which contains any selected from the group consisting of a fucosylated human milk oligosaccharide, a sialylated human milk oligosaccharide, a human milk oligosaccharide containing lactose as a constituent sugar, a human milk oligosaccharide containing fucose as a constituent sugar, a human milk oligosaccharide containing sialic acid as a constituent sugar, lactose, sialic acid, and fucose; or the use in manufacture, method, or use according to 9, wherein the composition is such a composition.
[11] The composition according to 9 or 10, which contains any selected from the group consisting of 2'-fucosyllactose, 3'-sialyllactose, 6'-sialyllactose, lactose, N-acetylneuraminic acid, and fucose; or the use in manufacture, method, or use according to 9 or 10, wherein the composition is such a composition.
[12] The composition according to any one of 9 to 11, which is for use as a prebiotic or synbiotic; the use in manufacture according to any one of 9 to 11, wherein the composition is such a composition; the method according to any one of 9 to 11, which comprises the step of administering the composition as a prebiotic or synbiotic; or the use according to any one of 9 to 11, wherein the composition is used as a prebiotic or synbiotic.
[13] The composition according to any one of 9 to 12, which is for use in treating any selected from the group consisting of obesity, abnormal sugar metabolism, diabetes mellitus, cancer, and inflammatory bowel disease; the use in manufacture according to any one of 9 to 12, wherein the composition is such a composition; the method according to any one of 9 to 12, which is for treating any selected from the group consisting of obesity, abnormal sugar metabolism, diabetes mellitus, cancer, and inflammatory bowel disease; or the use according to any one of 9 to 12, which is for such a treatment.
[14] A composition for treating a disease or condition that can be ameliorated by controlling growth of bacteria of the genus *Blautia* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Blautia* in the intestinal tract, a method for treating a disease or condition that can be ameliorated by controlling growth of bacteria of the genus *Blautia* in the intestinal tract, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating a disease or condition that can be ameliorated by controlling growth of bacteria of the genus *Blautia* in the intestinal tract.
[15] A composition for treating any selected from the group consisting of obesity, abnormal sugar metabolism, diabetes mellitus, cancer, and inflammatory bowel disease, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating any selected from the group consisting of obesity, abnormal sugar metabolism, diabetes mellitus, cancer, and inflammatory bowel disease, a method for treating any selected from the group consisting of obesity, abnormal sugar metabolism, diabetes mellitus, cancer, and inflammatory bowel disease, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating any selected from the group consisting of obesity, abnormal sugar metabolism, diabetes mellitus, cancer, and inflammatory bowel disease.
[16] The composition, use in manufacture of a composition, method, or use according to any one of 9 to 14, wherein the control of growth is promotion of growth.

The present invention provides the followings.
[1] A composition for controlling growth of bacteria of the genus *Subdoligranulum* in the intestinal bacterial microbiota, which contains any selected from the group consisting of a human milk oligosaccharide and a constituent sugar of a human milk oligosaccharide.
[2] The composition according to 1, which contains any selected from the group consisting of a sialylated human milk oligosaccharide, a human milk oligosaccharide containing sialic acid as a constituent sugar, and sialic acid.
[3] The composition according to 1 or 2, which contains any selected from the group consisting of 3'-sialyllactose, 6'-sialyllactose, and N-acetylneuraminic acid.
[4] The composition according to any one of 1 to 3, which is for use as a prebiotic or synbiotic.
[5] The composition according to any one of 1 to 4, which is for use in treating any selected from the group consisting of obesity, abnormal lipid metabolism, insulin resistance, diabetes mellitus, food allergy, and inflammatory bowel disease.
[6] A composition for treating a disease or condition that can be ameliorated by controlling growth of bacteria of the genus *Subdoligranulum* in the intestinal tract, which contains any selected from the group consisting of a human milk oligosaccharide and a constituent sugar of a human milk oligosaccharide.
[7] A composition for treating any selected from the group consisting of obesity, abnormal lipid metabolism, insulin resistance, diabetes mellitus, food allergy, and inflammatory bowel disease, which contains any selected from the group consisting of a human milk oligosaccharide and a constituent sugar of a human milk oligosaccharide.
[8] The composition according to 7 or 8, which contains any selected from the group consisting of a sialylated human milk oligosaccharide, a human milk oligosaccharide containing sialic acid as a constituent sugar, and sialic acid.
[9] The composition according to any one of 1 to 7, wherein the control of growth is promotion of growth.
[10] A composition for use in a method for controlling growth of bacteria of the genus *Subdoligranulum* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of bacteria of the genus *Subdoligranulum* in the intestinal bacterial microbiota, a method for controlling growth of bacteria of the genus *Subdoligranulum* in the intestinal bacterial microbiota, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for controlling growth of bacteria of the genus *Subdoligranulum* in the intestinal bacterial microbiota.
[11] The composition according to 10, which contains any selected from the group consisting of a sialylated human milk oligosaccharide, a human milk oligosaccharide containing sialic acid as a constituent sugar, and sialic acid; or the use in manufacture, method, or use according to 10, wherein the composition is such a composition.
[12] The composition according to 10 or 11, which contains any selected from the group consisting of 3'-sialyllactose, 6'-sialyllactose, and N-acetylneuraminic acid; or the use in manufacture, method, or use according to 10 or 11, wherein the composition is such a composition.
[13] The composition according to any one of 10 to 12, which is for use as a prebiotic or synbiotic; the use in manufacture according to any one of 10 to 12, wherein the composition is such a composition; the method according to any one of 10 to 12, which comprises the step of administering the composition as a prebiotic or synbiotic; or the use of a composition according to any one of 10 to 12, wherein the composition is used as a prebiotic or synbiotic.
[14] The composition according to any one of 10 to 13, which is for use in treating any selected from the group consisting of obesity, abnormal lipid metabolism, insulin resistance, diabetes mellitus, food allergy, and inflammatory bowel disease; use in manufacture according to any one of 10 to 13, wherein the composition is such a composition; the method according to any one of 10 to 13, which comprises the step of administering the composition for treating any selected from the group consisting of obesity, abnormal lipid metabolism, insulin resistance, diabetes mellitus, food allergy, and inflammatory bowel disease; or the use of a composition according to any one of 10 to 13, which is for use in treating any selected from the group consisting of obesity, abnormal lipid metabolism, insulin resistance, diabetes mellitus, food allergy, and inflammatory bowel disease.
[15] A composition for treating a disease or condition that can be ameliorated by controlling growth of bacteria of the genus *Subdoligranulum* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Subdoligranulum* in the intestinal tract, a method for treating a disease or condition that can be ameliorated by controlling growth of bacteria of the genus *Subdoligranulum* in the intestinal tract, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating a disease or condition that can be ameliorated by controlling growth of bacteria of the genus *Subdoligranulum* in the intestinal tract.
[16] A composition for treating any selected from the group consisting of obesity, abnormal lipid metabolism, insulin resistance, diabetes mellitus, food allergy, and inflammatory bowel disease, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating any selected from the group consisting of obesity, abnormal lipid metabolism, insulin resistance, diabetes mellitus, food allergy, and inflammatory bowel disease, a method for treating any selected from the group consisting of obesity, abnormal lipid metabolism, insulin resistance, diabetes mellitus, food allergy, and inflammatory bowel disease, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating any selected from the group consisting of obesity, abnormal lipid metabolism, insulin resistance, diabetes mellitus, food allergy, and inflammatory bowel disease.
[17] The composition according to 15 or 16, which contains any selected from the group consisting of a sialylated human milk oligosaccharide, a human milk oligosaccharide containing sialic acid as a constituent sugar, and sialic acid; or the use in manufacture, method, or use according to 15 or 16, wherein the composition is such a composition.
[18] The composition, use in manufacture, method, or use according to any one of 10 to 15, wherein the control of growth is promotion of growth.

The present invention provides the followings.
[1] A composition for controlling growth of bacteria of the genus *Bilophila* in the intestinal bacterial microbiota, which contains any selected from the group consisting of a human milk oligosaccharide and a constituent sugar of a human milk oligosaccharide.
[2] The composition according to 1, which contains any selected from the group consisting of a sialylated human milk oligosaccharide, a human milk oligosaccharide containing sialic acid as a constituent sugar, and sialic acid.
[3] The composition according to 1 or 2, which contains any selected from the group consisting of 3'-sialyllactose and N-acetylneuraminic acid.
[4] The composition according to any one of 1 to 3, which is for use as a prebiotic or synbiotic.
[5] The composition according to any one of 1 to 5, which is for use in treating any selected from the group consisting of abnormal sugar metabolism, diabetes mellitus, obesity, cancer, appendicitis, inflammatory bowel disease, liver dysfunction, intestinal barrier dysfunction, and metabolic syndrome.
[6] A composition for treating a disease or condition that can be ameliorated by controlling growth of bacteria of the genus *Bilophila* in the intestinal tract, which contains any selected from the group consisting of a human milk oligosaccharide and a constituent sugar of a human milk oligosaccharide.
[7] A composition for treating any selected from the group consisting of abnormal sugar metabolism, diabetes mellitus, obesity, cancer, appendicitis, inflammatory bowel disease, liver dysfunction, intestinal barrier dysfunction, and metabolic syndrome, which contains any selected from the group consisting of a human milk oligosaccharide and a constituent sugar of a human milk oligosaccharide.
[8] The composition according to any one of 1 to 9, wherein the control of growth is suppression of growth.
[9] A composition for use in a method for controlling growth of bacteria of the genus *Bilophila* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of bacteria of the genus *Bilophila* in the intestinal bacterial microbiota, a method for controlling growth of bacteria of the genus *Bilophila* in the intestinal bacterial microbiota, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for controlling growth of bacteria of the genus *Bilophila* in the intestinal bacterial microbiota.
[10] The composition according to 9, which contains any selected from the group consisting of a sialylated human milk oligosaccharide, a human milk oligosaccharide containing sialic acid as a constituent sugar, and sialic acid; or the use in manufacture, method, or use according to 9, wherein the composition is such a composition.
[11] The composition according to 9 or 10, which contains any selected from the group consisting of 3'-sialyllactose and N-acetylneuraminic acid; or the use in manufacture, method, or use according to 9 or 10, wherein the composition is such a composition.
[12] The composition according to any one of 9 to 11, which is for use as a prebiotic or synbiotic; the use in manufacture according to any one of 9 to 11, wherein the composition is such a composition; the method according to any one of 9 to 11, which comprises the step of administering the composition as a prebiotic or synbiotic; or the use according to any one of 9 to 11, wherein the composition is used as a prebiotic or synbiotic.
[13] The composition according to any one of 9 to 12, which is for use in treating any selected from the group consisting of abnormal sugar metabolism, diabetes mellitus, obesity, cancer, appendicitis, inflammatory bowel disease, liver dysfunction, intestinal barrier dysfunction, and metabolic syndrome; the use in manufacture according to any one of 9 to 12, wherein the composition is such a composition; the method according to any one of 9 to 12, which is for treating any selected from the group consisting of abnormal sugar metabolism, diabetes mellitus, obesity, cancer, appendicitis, inflammatory bowel disease, liver dysfunction, intestinal barrier dysfunction, and metabolic syndrome; or the use according to any one of 9 to 12, which is for such a treatment.
[14] A composition for treating a disease or condition that can be ameliorated by controlling growth of bacteria of the genus *Bilophila* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Bilophila* in the intestinal tract, a method for treating a disease or condition that can be ameliorated by controlling growth of bacteria of the genus *Bilophila* in the intestinal tract, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating a disease or condition that can be ameliorated by controlling growth of bacteria of the genus *Bilophila* in the intestinal tract.
[15] A composition for treating any selected from the group consisting of abnormal sugar metabolism, diabetes mellitus, obesity, cancer, appendicitis, inflammatory bowel disease, liver dysfunction, intestinal barrier dysfunction, and metabolic syndrome, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating any selected from the group consisting of abnormal sugar metabolism, diabetes mellitus, obesity, cancer, appendicitis, inflammatory bowel disease, liver dysfunction, intestinal barrier dysfunction, and metabolic syndrome, a method for treating any selected from the group consisting of abnormal sugar metabolism, diabetes mellitus, obesity, cancer, appendicitis, inflammatory bowel disease, liver dysfunction, intestinal barrier dysfunction, and metabolic syndrome, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating any selected from the group consisting of abnormal sugar metabolism, diabetes mellitus, obesity, cancer, appendicitis, inflammatory bowel disease, liver dysfunction, intestinal barrier dysfunction, and metabolic syndrome.
[16] The composition, use in manufacture of a composition, method, or use according to any one of 9 to 14, wherein the control of growth is suppression of growth.

The present invention provides the followings.
[1] A composition for controlling growth of bacteria of the genus *Sutterella* in the intestinal bacterial microbiota, which contains any selected from the group consisting of a human milk oligosaccharide and a constituent sugar of a human milk oligosaccharide.
[2] The composition according to 1, which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid.
[3] The composition according to 1 or 2, which contains any selected from the group consisting of 2'-fucosyllactose, 3'-sialyllactose, lactose, and N-acetylneuraminic acid.
[4] The composition according to any one of 1 to 3, which is for use as a prebiotic or synbiotic.
[5] The composition according to any one of 1 to 4, which is for use in treating any selected from the group consisting of developmental disorder and metabolic syndrome.
[6] A composition for treating a disease or condition that can be ameliorated by controlling growth of bacteria of the genus *Sutterella* in the intestinal tract, which contains any selected from the group consisting of a human milk oligosaccharide and a constituent sugar of a human milk oligosaccharide.
[7] A composition for treating any selected from the group consisting of developmental disorder and metabolic syndrome, which contains any selected from the group consisting of a human milk oligosaccharide and a constituent sugar of a human milk oligosaccharide.
[8] The composition according to any one of 1 to 6, wherein the control of growth is suppression of growth.
[9] A composition for use in a method for controlling growth of bacteria of the genus *Sutterella* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of bacteria of the genus *Sutterella* in the intestinal bacterial microbiota, a method for controlling growth of bacteria of the genus *Sutterella* in the intestinal bacterial microbiota, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for controlling growth of bacteria of the genus *Sutterella* in the intestinal bacterial microbiota.
[10] The composition according to 9, which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid; or the use in manufacture, method, or use according to 9, wherein the composition is such a composition.
[11] The composition according to 9 or 10, which contains any selected from the group consisting of 2'-fucosyllactose, 3'-sialyllactose, lactose, and N-acetylneuraminic acid; or the use in manufacture, method, or use according to 9 or 10, wherein the composition is such a composition.
[12] The composition according to any one of 9 to 11, which is for use as a prebiotic or synbiotic; the use in manufacture according to any one of 9 to 11, wherein the composition is such a composition; the method according to any one of 9 to 11, which comprises the step of administering the composition as a prebiotic or synbiotic; or the use according to any one of 9 to 11, wherein the composition is used as a prebiotic or synbiotic.
[13] The composition according to any one of 9 to 12, which is for use in treating any selected from the group consisting of developmental disorder and metabolic syndrome; the use in manufacture according to any one of 9 to 12, wherein the composition is such a composition; the method according to any one of 9 to 12, which is for treating any selected from the group consisting of developmental disorder and metabolic syndrome; or the use according to any one of 9 to 12, which is for such treatment.
[14] A composition for treating a disease or condition that can be ameliorated by controlling growth of bacteria of the genus *Sutterella* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Sutterella* in the intestinal tract, a method for treating a disease or condition that can be ameliorated by controlling growth of bacteria of the genus *Sutterella* in the intestinal tract, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating a disease or condition that can be ameliorated by controlling growth of bacteria of the genus *Sutterella* in the intestinal tract.
[15] A composition for treating any selected from the group consisting of developmental disorder and metabolic syndrome, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating any selected from the group consisting of developmental disorder and metabolic syndrome, a method for treating any selected from the group consisting of developmental disorder and metabolic syndrome, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating any selected from the group consisting of developmental disorder and metabolic syndrome.
[16] The composition, use in manufacture of a composition, method, or use according to any one of 9 to 14, wherein the control of growth is suppression of growth.

### Effects of the Invention

According to the present invention, the intestinal bacterial microbiota can be controlled. In particular, growth of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota can be controlled.

It can be expected that, through controlling increase of bacteria of the genus *Faecalibacterium* in the intestinal bacterial microbiota with specific active ingredients, any selected from the group consisting of irritable bowel syndrome, inflammatory bowel disease, Parkinson's disease, cancer, cognitive decline, and atopic dermatitis can be treated, and various diseases or conditions that can be ameliorated by controlling increase of bacteria of the genus *Faecalibacterium* in the intestinal bacterial microbiota can also be treated. An anti-inflammatory composition can also be provided by the present invention.

It can be expected that, through controlling increase of bacteria of the genus *Akkermansia* in the intestinal bacterial microbiota with specific active ingredients, any selected from the group consisting of obesity, abnormal sugar metabolism, inflammatory bowel disease, autism, and atopic dermatitis can be treated, and various diseases or conditions that can be ameliorated by controlling increase of bacteria of the genus *Akkermansia* in the intestinal bacterial microbiota can also be treated.

It can be expected that, through controlling increase of bacteria of the genus *Blautia* in the intestinal bacterial microbiota with specific active ingredients, any selected from the group consisting of obesity, abnormal sugar metabolism, diabetes mellitus, cancer, and inflammatory bowel disease can be treated, and various diseases or conditions that can be ameliorated by controlling increase of bacteria of the genus *Blautia* in the intestinal bacterial microbiota can also be treated.

It can be expected that, through controlling increase of bacteria of the genus *Subdoligranulum* in the intestinal bacterial microbiota with specific active ingredients, any selected from the group consisting of obesity, abnormal lipid metabolism, insulin resistance, diabetes mellitus, food allergy, and inflammatory bowel disease can be treated, and various diseases or conditions that can be ameliorated by controlling increase of bacteria of the genus *Subdoligranulum* in the intestinal bacterial microbiota can also be treated.

It can be expected that, through controlling growth or increase in occupancy rate of bacteria of the genus *Bilophila* in the bacterial microbiota with specific active ingredients, any selected from the group consisting of abnormal sugar metabolism, diabetes mellitus, obesity, cancer, appendicitis, inflammatory bowel disease, liver dysfunction, intestinal barrier dysfunction, and metabolic syndrome can be treated, and various diseases or conditions that can be ameliorated by controlling increase of bacteria of the genus *Bilophila* in the intestinal bacterial microbiota can also be treated.

According to the present invention, it can be expected that, through controlling growth or increase in occupancy rate of bacteria of the genus *Sutterella* in bacterial microbiota with specific active ingredients, any selected from the group consisting of developmental disorder and metabolic syndrome can be treated, and various diseases or conditions that can be ameliorated by controlling increase of bacteria of the genus *Sutterella* in the intestinal bacterial microbiota can also be treated.

By adding the active ingredients of the present invention, functional food products in various forms can be provided.

### Brief Description of the Drawings

[Fig. 1] Changes in occupancy rate of bacteria of the genus *Faecalibacterium* induced by various carbohydrates in a human fecal fermentation system, evaluated by the paired t-test.
[Fig. 2] Changes in occupancy rate of bacteria of the genus *Akkermansia* induced by various carbohydrates in a human fecal fermentation system, evaluated by the Wilcoxon signed rank test.
[Fig. 3] Changes in occupancy rate of bacteria of the genus *Blautia* induced by various carbohydrates in a human fecal fermentation system, evaluated by the Wilcoxon signed rank test.
[Fig. 4] Changes in occupancy rate of bacteria of the genus *Subdoligranulum* in a human fecal fermentation system induced by various carbohydrates, evaluated by the Wilcoxon signed rank test or paired t-test.
[Fig. 5] Changes in occupancy rate of bacteria of the genus *Bilophila* induced by various carbohydrates in a human fecal fermentation system. The symbol * indicates P <0.001 versus control, evaluated by the Dunnett's multiple comparisons test.
[Fig. 6] Changes in occupancy rate of bacteria of the genus *Sutterella* induced by various carbohydrates in a human fecal fermentation system, evaluated by the Wilcoxon signed rank test or paired t-test.

### Modes for Carrying out the Invention

Hereafter, the present invention will be explained in detail.

The present invention relates to a composition for controlling growth of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, which contains any selected from the group consisting of a human milk oligosaccharide (also referred to as HMO) and a constituent sugar of a human milk oligosaccharide as an active ingredient.

### [Active Ingredient]

The composition of the present invention contains any selected from the group consisting of a human milk oligosaccharide (also referred to as HMO) and a constituent sugar of a human milk oligosaccharide as an active ingredient. Human milk oligosaccharides are oligosaccharides contained in human milk and are the third most abundant solid component after lactose and lipids. With few exceptions, human milk oligosaccharides have a chemical structure comprising a lactose unit at the reducing end, to which fucose, sialic acid, galactose and so forth are added.

For the present invention, the abbreviation and structure of milk oligosaccharides are according to the descriptions of Nutr Rev. 2017 Nov 1;75(11):920-933. doi: 10.1093/nutrit/nux044; and Nutrients 2021, 13(8), 2737; https://doi.org/10.3390/nu13082737. In case of discrepancies between these references, the latter should be followed.

The human milk oligosaccharide and constituent sugar of a human milk oligosaccharide used in the composition are not particularly limited so long as they can provide the desired effect. The human milk oligosaccharide and constituent sugar of a human milk oligosaccharide used in the composition may consist of one type or a combination of two or more types thereof.

### Examples of the human milk oligosaccharide contained in the composition include the following neutral and acidic oligosaccharides

Neutral oligosaccharides: 2'-fucosyllactose (2'-FL), 3-fucosyllactose (3-FL), difucosyllactose (DFL), lacto-difucotetraose (LDFT), lacto-N-tetraose (LNT), lacto-N-neotetraose (LNnT), lacto-N-fucopentaose I (LNFP I), lacto-N-fucopentaose II (LNFP II), lacto-N-fucopentaose III (LNFP III), lacto-N-fucopentaose V (LNFP V), lacto-N-fucopentaose VI (LNFP VI), lacto-N-difucohexaose I (LNDFH I), lacto-N-difucohexaose II (LNDFH II), lacto-N-hexaose (LNH), para-lacto-N-hexaose (pLNH), lacto-N-neohexaose (LNnH), para-lacto-N-neohexaose (pLNnH), fucosyllacto-N-hexaose I (F-LNH I), fucosyl-para-lacto-N-hexaose I (F-para-LNH I), fucosyllacto-N-hexaose II (F-LNH II), fucosyllacto-N-hexaose III (F-LNH III), difucosyllacto-N-hexaose I (DF-LNH I), difucosyllacto-N-hexaose II (DF-LNH II), difucosyllacto-N-hexaose III (DF-LNH III), difucosyl-para-lacto-N-neohexaose (DF -pLNnH), difucosyl-para-lacto-N-hexaose (DF-para-LNH), difucosyl-para-lacto-N-neohexaose (DF-para-LNnH), and trifucosyllacto-N-hexaose (TF-LNH).

Acidic oligosaccharides: 3'-sialyllactose (3'-SL), 6'-sialyllactose (6'-SL), 6'-sialyl-N-acetyllactosamine (6'-SLN), LS-tetrasaccharide a (LST a, sialyllacto-N-tetraose a), LS-tetrasaccharide b (LST b, sialyllacto-N-tetraose b), LS-tetrasaccharide c (LST c, sialyllacto-N-tetraose c), disialyllacto-N-tetraose (DS-LNT), fucosyl-sialyllacto-N-tetraose a (F-LST a), fucosyl-sialyllacto-N-tetraose b (F-LST b), fucosyl-sialyllacto-N-hexaose (FS-LNH), fucosyl-sialyllacto-N-neohexaose (FS-LNnH I), fucosyl-disialyllacto-N-hexaose (FDS-LNH), and disialyllacto-N-hexaose (DS-LNH).

Preferred examples of the human milk oligosaccharide contained in the composition include the following neutral and acidic oligosaccharides. Neutral oligosaccharides: 2'-fucosyllactose (2'-FL), 3-fucosyllactose (3-FL), difucosyllactose (DFL), lacto-difucotetraose (LDFT), lacto-N-tetraose (LNT), lacto-N-neotetraose (LNnT), lacto-N-fucopentaose I (LNFP I), lacto-N-fucopentaose II (LNFP II), lacto-N-fucopentaose III (LNFP III), lacto-N-fucopentaose V (LNFP V), lacto-N-fucopentaose VI (LNFP VI), lacto-N-difucohexaose I (LNDFH I), lacto-N-difucohexaose II (LNDFH II), lacto-N-hexaose (LNH), lacto-N-neohexaose (LNnH), para-lacto-N-neohexaose (pLNnH), fucosyllacto-N-hexaose I (F-LNH I), fucosyl-para-lacto-N-hexaose I (F-para-LNH I), fucosyllacto-N-hexaose II (F-LNH II), fucosyllacto-N-hexaose III (F-LNH III), difucosyllacto-N-hexaose I (DF-LNH I), difucosyllacto-N-hexaose II (DF-LNH II), difucosyllacto-N-hexaose III (DF-LNH III), difucosyl-para-lacto-N-neohexaose (DF-pLNnH), and trifucosyllacto-N-hexaose (TF-LNH).

Acidic oligosaccharides: 3'-sialyllactose (3'-SL), 6'-sialyllactose (6'-SL), LS-tetrasaccharide a (LST a, sialyllacto-N-tetraose a), LS-tetrasaccharide b (LST b, sialyllacto-N-tetraose b), LS-tetrasaccharide c (LST c, sialyllacto-N-tetraose c), disialyllacto-N-tetraose (DS-LNT), fucosyl-sialyllacto-N-neohexaose (FS-LNnH I), and disialyllacto-N-hexaose (DS-LNH).

More preferred examples of the human milk oligosaccharide contained in the composition include the following neutral and acidic oligosaccharides. Neutral oligosaccharides: 2'-fucosyllactose (2'-FL), difucosyllactose (DFL), lacto-difucotetraose (LDFT), lacto-N-tetraose (LNT), and lacto-N-neotetraose (LNnT). Acidic oligosaccharides: 3'-sialyllactose (3'-SL) and 6'-sialyllactose (6'-SL)

For the present invention, the term constituent sugar of a human milk oligosaccharide refers to a sugar constituting a human milk oligosaccharide (sugar resulting from degradation of human milk oligosaccharide), excluding glucose and galactose. Examples of the constituent sugar of a human milk oligosaccharide contained in the composition include lactose, fucose, sialic acid, and N-acetylglucosamine. Sialic acid is a general term for modifications of neuraminic acid. An example of sialic acid is N-acetylneuraminic acid (Neu5Ac), which is an acetyl-modified neuraminic acid.

According to the studies of the inventors of the present invention, there were confirmed superior growth-promoting effects of lactose, Neu5Ac, fucose, 2'-FL, 3'-SL, and 6'-SL for bacteria of the genera *Faecalibacterium, Akkermansia,* and *Blautia.* By the way, human milk oligosaccharides can generate lactose in the human digestive tract after ingestion. In addition, human milk oligosaccharides containing Neu5Ac and human milk oligosaccharides containing fucose as a constituent sugar can generate Neu5Ac and fucose, respectively, in the human digestive tract after ingestion (see Non-patent documents 14 to 17 mentioned below). Furthermore, since superior growth-promoting effects for bacteria of the genera *Faecalibacterium, Akkermansia,* and *Blautia* were confirmed for all of 2'-FL, 3'-SL, and 6'-SL, the desired effect would be sufficiently obtained with fucosylated human milk oligosaccharides and sialylated human milk oligosaccharides.

From the viewpoint of promoting growth of bacteria of the genera *Faecalibacterium, Akkermansia,* and *Blautia,* preferred examples of the human milk oligosaccharide and constituent sugar of a human milk oligosaccharide used in the composition are any selected from the group consisting of fucosylated human milk oligosaccharides, sialylated human milk oligosaccharides, human milk oligosaccharides containing lactose as a constituent sugar, human milk oligosaccharides containing fucose as a constituent sugar, human milk oligosaccharides containing sialic acid as a constituent sugar, lactose, sialic acid, and fucose. Human milk oligosaccharides containing fucose as a constituent sugar include fucosylated human milk oligosaccharides (e.g., 2'-FL) and fucose-containing human milk oligosaccharides (e.g., DFL (LDFT)). Human milk oligosaccharides containing sialic acid as a constituent sugar include sialylated human milk oligosaccharides (e.g., 3'-SL and 6'-SL) and sialic acid-containing human milk oligosaccharides (e.g., LST a and DS-LNT).

From the viewpoint of promoting growth of bacteria of the genera *Faecalibacterium, Akkermansia,* and *Blautia,* specific examples of the human milk oligosaccharide and constituent sugar of a human milk oligosaccharide used in the composition are any selected from the group consisting of 2'-FL, 3'-SL, 6'-SL, lactose, Neu5Ac, and fucose.

From the viewpoint of promoting growth of bacteria of the genera *Faecalibacterium, Akkermansia,* and *Blautia,* other preferred example of the human milk oligosaccharide and constituent sugar of a human milk oligosaccharide used in the composition are any selected from the group consisting of sialylated human milk oligosaccharides, human milk oligosaccharides containing lactose as a constituent sugar, human milk oligosaccharides containing sialic acid as a constituent sugar, lactose, sialic acid, and fucose. Human milk oligosaccharides containing sialic acid as a constituent sugar include sialylated human milk oligosaccharides (e.g., 3'-SL and 6'-SL) and sialic acid-containing human milk oligosaccharides (e.g., LST a and DS-LNT). Specific examples are any selected from the group consisting of 3'-SL, 6'-SL, lactose, Neu5Ac, and fucose.

According to the studies of the inventors of the present invention, there have been confirmed superior growth-promoting effects of Neu5Ac, 3'-SL, and 6'-SL for bacteria of the genus *Subdoligranulum.* Concerning the above, human milk oligosaccharides containing Neu5Ac as a constituent sugar can generate Neu5Ac in the human digestive tract after ingestion (see Non-patent documents 14 to 17 mentioned later). Therefore, human milk oligosaccharides containing sialic acid as a constituent sugar and sialylated human milk oligosaccharides would sufficiently provide the desired growth-promoting effect for bacteria of the genus *Subdoligranulum.*

From the viewpoint of promoting growth of bacteria of the genus *Subdoligranulum* bacteria, preferred examples of the human milk oligosaccharide and constituent sugar of a human milk oligosaccharide used in the composition are any selected from the group consisting of sialylated human milk oligosaccharides, human milk oligosaccharides containing sialic acid as a constituent sugar, and sialic acid. Human milk oligosaccharides containing sialic acid as a constituent sugar include sialylated human milk oligosaccharides (e.g., 3'-SL and 6'-SL) and sialic acid-containing human milk oligosaccharides (e.g., LST a and DS-LNT).

From the viewpoint of promoting growth of bacteria of the genus *Subdoligranulum,* specific examples of the human milk oligosaccharide and constituent sugar of a human milk oligosaccharide used in the composition include any selected from the group consisting of Neu5Ac, 3'-SL, and 6'-SL.

According to the studies of the inventors of the present invention, there have been confirmed superior growth-suppressing effect of Neu5Ac and 3'-SL for bacteria of the genus *Bilophila.* Concerning the above, human milk oligosaccharides containing Neu5Ac as a constituent sugar can generate Neu5Ac in the human digestive tract after ingestion (see Non-patent document 14 to 17 mentioned later). Furthermore, since the desired growth-suppressing effect for bacteria of the genus *Bilophila* was confirmed for 3'-SL, sialylated human milk oligosaccharides would sufficiently provide the desired effect.

From the viewpoint of suppressing growth of bacteria of the genus *Bilophila,* preferred examples of the human milk oligosaccharide and constituent sugar of a human milk oligosaccharide used in the composition are any selected from the group consisting of sialylated human milk oligosaccharides, human milk oligosaccharides containing sialic acid as a constituent sugar, and sialic acid. Human milk oligosaccharides containing sialic acid as a constituent sugar include sialylated human milk oligosaccharides (e.g., 3'-SL) and sialic acid-containing human milk oligosaccharides (e.g., LST a and DS-LNT).

From the viewpoint of suppressing growth of bacteria of the genus *Bilophila,* specific examples of the human milk oligosaccharide and constituent sugar of a human milk oligosaccharide used in the composition are any selected from the group consisting of 3'-SL and Neu5Ac.

According to the studies of the inventors of the present invention, there were confirmed superior growth-suppressing effects of lactose, Neu5Ac, 2'-FL, and 3'-SL for bacteria of the genus *Sutterella.* Concerning the above, human milk oligosaccharides can generate lactose in the human digestive tract after ingestion. In addition, human milk oligosaccharides containing Neu5Ac as a constituent sugar can generate Neu5Ac in the human digestive tract after ingestion (see Non-patent documents 14 to 17 mentioned later). Furthermore, the desired effect was confirmed for 2'-FL and 3'-SL, and therefore fucosylated human milk oligosaccharides and sialylated human milk oligosaccharides would sufficiently provide the desired growth-suppressing effect for bacteria of the genus *Sutterella.*

From the viewpoint of suppressing growth of bacteria of the genus *Sutterella,* preferred examples of the human milk oligosaccharide and constituent sugar of a human milk oligosaccharide used in the composition are any selected from the group consisting of fucosylated human milk oligosaccharides, sialylated human milk oligosaccharides, human milk oligosaccharides containing lactose as a constituent sugar, human milk oligosaccharides containing sialic acid as a constituent sugar, lactose, and sialic acid. Human milk oligosaccharides containing sialic acid as a constituent sugar include sialylated human milk oligosaccharides (e.g., 3'-SL) and sialic acid-containing human milk oligosaccharides (e.g., LST a and DS-LNT).

From the viewpoint of suppressing growth of bacteria of the genus *Sutterella,* specific examples of the human milk oligosaccharide and constituent sugar of a human milk oligosaccharide used in the composition are any selected from the group consisting of 2'-FL, 3'-SL, lactose, and Neu5Ac.

### [Use]

The composition of the present invention can be used to control growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in intestinal bacterial microbiota. The control of growth includes promotion of growth and suppression of growth. Preferred compositions are for promotion of growth for any bacteria selected from the group consisting of bacteria of the genus *Faecalibacterium,* bacteria of the genus *Akkermansia,* bacteria of the genus *Blautia,* and bacteria of the genus *Subdoligranulum,* and are for suppression of growth for any bacteria selected from the group consisting of bacteria of the genus *Bilophila* and bacteria of the genus *Sutterella.*

Examples of bacteria of the intestinal bacterial microbiota include the followings:
bacteria of the genus *Faecalibacterium,* e.g., *Faecalibacterium prausnitzii;*
bacteria of the genus *Akkermansia,* e.g., *Akkermansia muciniphila;*
bacteria of the genus *Blautia,* e.g., *Blautia coccoides, Blautia intestinalis,* and *Blautia faecicola;*
bacteria of the genus *Subdoligranulum,* e.g., *Subdoligranulum variabile;*
bacteria of the genus *Bilophila* e.g., *Bilophila wadsworthia;*
bacteria of the genus *Collinsella,* e.g., *Collinsella aerofaciens* and *Collinsella intestinalis;*
bacteria of the genus *Holdemania,* e.g., *Holdemaniafiliformis;*
bacteria of the genus *Parasutterella,* e.g., *Parasutterella excrementihominis* and *Parasutterella secunda;*
bacteria of the genus *Oscillibacter,* e.g., *Oscillibacter valericigenes;*
bacteria of the genus *Eggerthella,* e.g., *Eggerthella lenta;*
bacteria of the genus *Sutterella,* e.g., *Sutterella parvirubra* and *Sutterella wadsworthensis;* and
bacteria of the genus *Bifidobacterium* (bifidobacteria), e.g., *B. adolescentis, B. bifidum, B. breve, B. catenulatum, B. infantis, B. lactis, B. longum,* and *B. pseudocatenulatum.*

For the present invention, the term bacteria of the genus *Faecalibacterium* refers to bacteria identified as belonging to the genus *Faecalibacterium* by molecular phylogenetic analysis based on the 16S rRNA gene. For the present invention, the term bacteria of the genus *Akkermansia* refers to bacteria identified as belonging to the genus *Akkermansia* by molecular phylogenetic analysis based on the 16S rRNA gene. For the present invention, the term bacteria of the genus *Blautia* refers to bacteria identified as belonging to the genus *Blautia* by molecular phylogenetic analysis based on the 16S rRNA gene. For the present invention, the term bacteria of the genus *Subdoligranulum* refers to bacteria identified as belonging to the genus *Subdoligranulum* by molecular phylogenetic analysis based on the 16S rRNA gene. For the present invention, the term bacteria of the genus *Bilophila* refers to bacteria identified as belonging to the genus *Bilophila* by molecular phylogenetic analysis based on the 16S rRNA gene. For the present invention, the term bacteria of the genus *Sutterella* refers to bacteria identified as belonging to the genus *Sutterella* by molecular phylogenetic analysis based on the 16S rRNA gene. Criteria for determining genus by molecular phylogenetic analysis based on the 16S rRNA gene are well known by those skilled in the art (Stackebrandt E, Ebers J. Taxonomic parameters revisited: tarnished gold standards. Microbiol Today. 2006;33:152-155).

For the present invention, controlling growth of specific bacteria in bacterial microbiota means controlling the proportion (occupancy rate) of the specific bacteria in the bacterial microbiota.

Whether or not a certain ingredient controls growth of a particular type of bacteria in the intestinal bacterial microbiota can be evaluated as follows. A fecal material provided by a healthy person is added to an appropriate medium, cultured for a certain period of time as required, then an ingredient to be evaluated is added, cultured under appropriate conditions (e.g., at 37°C under anaerobic conditions, similar to those in the intestinal tract, for 48 hours), and the occupancy rate of the particular type of bacteria in the bacterial microbiota in the culture is measured. The result of the measurement can then be compared with a measurement result of a culture obtained under conditions that differ only in that a control (e.g., sterile water) is added in place of the ingredient to be evaluated but that are otherwise identical, and it can be determined that, if the occupancy rate is higher than the control, growth is promoted, and if lower, growth is suppressed.

Occupancy rate of a particular type of bacteria in bacterial microbiota can be determined by known methods. One preferred method is to perform 16S metagenomic analysis (sequence analysis of 16S rRNA gene amplicons) on DNAs extracted from culture. When the 16S metagenomic analysis is performed, DNA extraction can be performed by using commercially available kits. The genomic region to be analyzed is not particularly limited so long as the bacteria can be determined, but the V3-V4 region of the 16S rRNA gene can be used. Primers, amplification conditions, method for purification of amplicons, and so forth well known to those skilled in the art can be used to analyze bacteria. Sequence decoding is preferably performed on a next-generation sequencer of higher performance. A next-generation microbiome bioinformatics platform such as QIIME 2^{™} can be used to analyze the obtained data. For the 16S metagenomic analysis, those skilled in the art can refer to such information as Sanschagrin S, Yergeau E. Next-generation sequencing of 16S ribosomal RNA gene amplicons. J Vis Exp. 2014;(90):51709. Published 2014 Aug 29. doi:10.3791/51709.

Bacteria of the genus *Faecalibacterium* as one of the objects of the present invention are predominant bacteria that account for about 5% of the total intestinal bacteria in healthy adults, and recently attract attention as bacterial species having favorable physiological functions for human health, such as butyrate production and anti-inflammatory activity. It has been revealed that bacteria of the genus *Faecalibacterium* are reduced in the intestines of patients of irritable bowel syndrome (IBS) (Gut Microbiota in Patients With Irritable Bowel Syndrome-A Systematic Review. Gastroenterology. 2019;157(1):97-108. doi:10.1053/j.gastro.2019.03.049), inflammatory bowel disease (IBD) (A decrease of the butyrate-producing species Roseburia hominis and *Faecalibacterium prausnitzii* defines dysbiosis in patients with ulcerative colitis. Gut. 2014;63(8):1275-1283. doi:10.1136/gutjnl-2013-304833), Parkinson's disease (Alterations of gut microbiota and metabolome with Parkinson's disease. Microb Pathog. 2021;160:105187), and cancer (The gut microbial diversity of colon cancer patients and the clinical significance. Bioengineered. 2021;12(1):7046-7060) compared with healthy individuals. It has also been reported that administration of bacteria of this genus to IBD model mice improves such symptoms as weight loss and diarrhea (Administration of live, but not inactivated, *Faecalibacterium prausnitzii* has a preventive effect on dextran sodium sulfate induced colitis in mice. Mol Med Rep. 2019;20(1):25-32), and administration of bacteria of this genus improves cognitive dysfunction in Alzheimer's disease model mice (Identification of *Faecalibacterium prausnitzii* strains for gut microbiome-based intervention in Alzheimer's-type dementia. Cell Rep Med. 2021;2(9): 100398. Published 2021 Sep 14). Furthermore, it has been reported that *Faecalibacterium prausnitzii* exhibited anti-inflammatory effects in cultured cell lines and in TNBS colitis model mice (*Faecalibacterium prausnitzii* is an anti-inflammatory commensal bacterium identified by gut microbiota analysis of Crohn disease patients. Proc Natl Acad Sci U S A. 2008;105(43):16731-16736. doi:10.1073/pnas.0804812105). Therefore, the composition can be used for treating any selected from the group consisting of irritable bowel syndrome, inflammatory bowel disease, Parkinson's disease, cancer, cognitive decline, and atopic dermatitis. The composition can also be used for anti-inflammatory purposes.

In addition, there is a report on the effects of 3'-SL or 6'-SL administration on bacterial species belonging to the phylum *Faecalibacterium* in the proximal and distal colons of porcine neonates (The human milk oligosaccharide 2'-fucosyllactose augments the adaptive response to extensive intestinal. Am J Physiol Gastrointest Liver Physiol. 2016 Mar 15; 310(6): G427-G438), but pigs and humans have different intestinal bacterial microbiota (Moor J, Wuthrich T, Aebi S, et al. Influence of pig farming on human Gut Microbiota: role of airborne microbial communities. Gut Microbes. 2021;13(1):1-13. doi:10.1080/19490976.2021.1927634).

Bacteria of the genus *Akkermansia,* especially *A. muciniphila,* as one of the objects of the present invention are bacteria that inhabit the intestines of humans and other mammals. It is known that decrease in *A. muciniphila* in humans is associated with such diseases as obesity, type 2 diabetes mellitus, inflammatory bowel disease (IBD), autism and atopy (Everard A, Belzer C, Geurts L, et al. Cross-talk between Akkermansia muciniphila and intestinal epithelium controls diet-induced obesity. Proc Natl Acad Sci USA. 2013;110(22):9066-9071. doi:10.1073/pnas.1219451110; Zhang T, Li P, Wu X, et al. Alterations of Akkermansia muciniphila in the inflammatory bowel disease patients with washed microbiota transplantation. Appl Microbiol Biotechnol. 2020;104(23): 10203-10215. doi:10.1007/s00253-020-10948-7; Zou R, Xu F, Wang Y, Duan M, Guo M, Zhang Q, Zhao H, Zheng H. Changes in the Gut Microbiota of Children with Autism Spectrum Disorder. Autism Res. 2020 Sep;13(9):1614-1625. doi: 10.1002/aur.2358. Epub 2020 Aug 24. PMID: 32830918; and Candela M, Rampelli S, Turroni S, Severgnini M, Consolandi C, De Bellis G, Masetti R, Ricci G, Pession A, Brigidi P. Unbalance of intestinal microbiota in atopic children. BMC Microbiol. 2012 Jun 6;12:95. doi: 10.1186/1471-2180-12-95. PMID: 22672413; PMCID: PMC3404014). It is also known that administration of *A. muciniphila* to mice ameliorates glucose metabolism and IBD (Plovier H, Everard A, Druart C, et al. A purified membrane protein from Akkermansia muciniphila or the pasteurized bacterium improves metabolism in obese and diabetic mice. Nat Med. 2017;23(1):107-113. doi:10.1038/nm.4236; and Bian X, Wu W, Yang L, et al. Administration of Akkermansia muciniphila Ameliorates Dextran Sulfate Sodium-Induced Ulcerative Colitis in Mice. Front Microbiol. 2019;10:2259. Published 2019 Oct 1. doi:10.3389/fmicb.2019.02259). Furthermore, it has been recognized that administration of *A. muciniphila* to humans provides metabolism-improving effects such as effects of improving insulin sensitivity and decreasing total cholesterol (Depommier C, Everard A, Druart C, Plovier H, Van Hul M, Vieira-Silva S, Falony G, Raes J, Maiter D, Delzenne NM, de Barsy M, Loumaye A, Hermans MP, Thissen JP, de Vos WM, Cani PD. Supplementation with Akkermansia muciniphila in overweight and obese human volunteers: a proof-of-concept exploratory study. Nat Med. 2019 Jul;25(7):1096-1103. doi: 10.1038/s41591-019-0495-2. Epub 2019 Jul 1. PMID: 31263284; PMCID: PMC6699990). Therefore, the composition can be used for treating any selected from the group consisting of obesity, abnormal sugar metabolism, inflammatory bowel disease, autism, and atopic dermatitis.

Bacteria of the genus *Blautia* as one of the objects of the present invention constitute one class of the predominant bacterial species accounting for about 5% of the total intestinal bacteria in healthy adults, especially in Japanese, 16% of the total intestinal bacteria (Nishijima S, Suda W, Oshima K, et al. The gut microbiome of healthy Japanese and its microbial and functional uniqueness. DNA Res. 2016;23(2):125-133. doi:10.1093/dnares/dsw002). It is also known that bacteria of the genus *Blautia* decrease in obese individuals, type 2 diabetics, elderly people, inflammatory bowel disease patients, and cancer patients (Benitez-Paez A, Gomez Del Pugar EM, Lopez-Almela I, Moya-Perez A, Codoner-Franch P, Sanz Y. Depletion of Blautia Species in the Microbiota of Obese Children Relates to Intestinal Inflammation and Metabolic Phenotype Worsening. mSystems. 2020;5(2):e00857-19. Published 2020 Mar 24. doi:10.1128/mSystems.00857-19; Inoue R, Ohue-Kitano R, Tsukahara T, et al. Prediction of functional profiles of gut microbiota from 16S rRNA metagenomic data provides a more robust evaluation of gut dysbiosis occurring in Japanese type 2 diabetic patients. J Clin Biochem Nutr. 2017;61(3):217-221. doi: 10.3 1 64/jcbn. 17-44; Rondanelli M, Giacosa A, Faliva MA, Perna S, Allieri F, Castellazzi AM. Review on microbiota and effectiveness of probiotics use in older. World J Clin Cases. 2015;3(2):156-162. doi:10.12998/wjcc.v3.i2.156; Takahashi K, Nishida A, Fujimoto T, et al. Reduced Abundance of Butyrate-Producing Bacteria Species in the Fecal Microbial Community in Crohn's Disease [published correction appears in Digestion. 2016;93(2):174]. Digestion. 2016;93(1):59-65. doi:10.1159/000441768; Chen W, Liu F, Ling Z, Tong X, Xiang C. Human intestinal lumen and mucosa-associated microbiota in patients with colorectal cancer. PLoS One. 2012;7(6):e39743. doi:10.1371/journal.pone.0039743; and Liu F, Li J, Guan Y, et al. Dysbiosis of the Gut Microbiome is associated with Tumor Biomarkers in Lung Cancer. Int J Biol Sci. 2019;15(11):2381-2392. Published 2019 Sep 7. doi:10.7150/ijbs.35980), and it is also known that they have anti-inflammatory action (Benitez-Paez A. et al., 2020 mentioned above). Therefore, the composition can be used for treating any selected from the group consisting of obesity, abnormal sugar metabolism, diabetes mellitus, cancer, and inflammatory bowel disease.

Bacteria of the genus *Subdoligranulum* as one of the objects of the present invention are bacteria that inhabit the intestines of humans and other mammals. They are known to be decreased common in children with food allergy and inflammatory bowel disease patients (Abdel-Gadir A, Stephen-Victor E, Gerber GK, et al. Microbiota therapy acts via a regulatory T cell MyD88/RORyt pathway to suppress food allergy [published correction appears in Nat Med. 2019 Sep;25(9):1458]. Nat Med, 2019;25(7):1164-1174. doi:10.103 8/s41591-019-0461-z; and Xia Y, Wang J, Fang X, Dou T, Han L, Yang C. Combined analysis of metagenomic data revealed consistent changes of gut microbiome structure and function in inflammatory bowel disease. J Appl Microbiol. 2021;131(6):3018-3031. doi:10.1111/jam.15154). It has also been shown that administration of bacteria of the genus *Subdoligranulum* to food allergy model mice can suppress their food allergy symptoms (Abdel-Gadir A. et. al., 2019 mentioned above), and furthermore, it is known that bacteria of the genus *Subdoligranulum* bacteria positively correlate with HDL cholesterol (Zhang X, Fang Z, Zhang C, et al. Effects of Acarbose on the Gut Microbiota of Prediabetic Patients: A Randomized, Double-blind, Controlled Crossover Trial. Diabetes Ther. 2017;8(2):293-307. doi:10.1007/s13300-017-0226-y), and that they negatively correlate with insulin resistance, body weight, and C-reactive protein (CRP), whose blood levels rise rapidly in the presence of inflammation and tissue destruction (Louis S, Tappu RM, Damms-Machado A, Huson DH, Bischoff SC. Characterization of the Gut Microbial Community of Obese Patients Following a Weight-Loss Intervention Using Whole Metagenome Shotgun Sequencing. PLoS One. 2016;11(2):e0149564. Published 2016 Feb 26. doi:10.1371/journal.pone.0149564). Therefore, the composition can be used for treating any selected from the group consisting of obesity, abnormal lipid metabolism, insulin resistance, diabetes mellitus, food allergy, and inflammatory bowel disease.

Since generation of hydrogen sulfide in the intestinal tract causes DNA damage, it has been suggested to be linked to cancer (Attene-Ramos MS, Nava GM, Muellner MG, Wagner ED, Plewa MJ, Gaskins HR. DNA damage and toxicogenomic analyses of hydrogen sulfide in human intestinal epithelial FHs 74 Int cells. Environ Mol Mutagen. 2010;51(4):304-314. doi:10.1002/em.20546) or inflammatory bowel disease caused by disruption of the intestinal barrier (Ij ssennagger N, van der Meer R, van Mil SWC. Sulfide as a Mucus Barrier-Breaker in Inflammatory Bowel Disease?. Trends Mol Med. 2016;22(3):190-199. doi:10.1016/j.molmed.2016.01.002). One source of hydrogen sulfide production in the intestinal tract is intestinal bacteria. Bacteria of the genus *Bilophila* constitute one class of hydrogen sulfide-producing bacteria in the intestinal tract, and have been suggested to implicate in appendicitis and colon cancer (Baron EJ, Curren M, Henderson G, et al. Bilophila wadsworthia isolates from clinical specimens. J Clin Microbiol. 1992;30(7):1882-1884. doi:10.1128/jcm.30.7.1882-1884.1992; and Yazici C, Wolf PG, Kim H, et al. Race-dependent association of sulfidogenic bacteria with colorectal cancer. Gut. 2017;66(11):1983-1994. doi:10.1136/gutjnl-2016-313321). It has also been reported that, in mice, bacteria of the genus *Bilophila* cause systemic inflammation (Feng Z, Long W, Hao B, et al. A human stool-derived Bilophila wadsworthia strain caused systemic inflammation in specific-pathogen-free mice. Gut Pathog. 2017;9:59. Published 2017 Oct 26. doi:10.1186/s13099-017-0208-7), and increase in the intestinal tract in association with worsening of colitis scores caused by high-fat diet (Devkota S, Wang Y, Musch MW, et al. Dietary-fat-induced taurocholic acid promotes pathobiont expansion and colitis in Il10-/- mice. Nature. 2012;487(7405):104-108. doi:10.1038/nature11225). In mice with high levels of *B. wadsworthia* in the intestinal tract, they synergistically with a high-fat diet (HFD) promotes inflammation, intestinal barrier dysfunction, and abnormal bile acid metabolism, leading to abnormal sugar metabolism and fatty liver (Natividad JM, Lamas B, Pham HP, et al. Bilophila wadsworthia aggravates high fat diet induced metabolic dysfunctions in mice. Nat Commun. 2018;9(1):2802. Published 2018 Jul 18. doi:10.1038/s41467-018-05249-7). Therefore, the composition of the present invention can be used for treating any selected from the group consisting of abnormal sugar metabolism, diabetes mellitus, obesity, cancer, appendicitis, inflammatory bowel disease, liver dysfunction, intestinal barrier dysfunction, and metabolic syndrome.

Bacteria of the genus *Sutterella* as an object of the present invention constitute one class of human intestinal bacteria, and have been reported to be associated with a variety of diseases. It has been reported that bacteria of the genus *Sutterella* are abundant in the intestines of children with autism spectrum disorder (Wang L, Christophersen CT, Sorich MJ, Gerber JP, Angley MT, Conlon MA. Increased abundance of Sutterella spp. and Ruminococcus torques in feces of children with autism spectrum disorder. Mol Autism. 2013;4(1):42. Published 2013 Nov 4. doi:10.1186/2040-2392-4-42), children with attention-deficit/hyperactivity disorder (ADHD) (Wang LJ, Yang CY, Chou WJ, et al. Gut microbiota and dietary patterns in children with attention-deficit/hyperactivity disorder. Eur Child Adolesc Psychiatry. 2020;29(3):287-297. doi:10.1007/s00787-019-01352-2), and those with metabolic syndrome (Lim MY, You HJ, Yoon HS, et al. The effect of heritability and host genetics on the gut microbiota and metabolic syndrome. Gut. 2017;66(6):1031-1038. doi:10.1136/gutjnl-2015-311326). Therefore, the composition can be used for treating any selected from the group consisting of developmental disorder and metabolic syndrome.

Irritable bowel syndrome (IBS) is a syndrome in which patients chronically complain of abdominal distention and abdominal pain, and experience abnormal bowel movements such as diarrhea and constipation, despite the absence of inflammation, ulceration, or endocrine abnormalities in the bowel on normal examination. It is believed to be caused by hypersensitivity of the visceral nerves of the intestines to stimuli due to mental stress or autonomic nervous system dysfunction. IBS includes those of chronic diarrheal, unstable, and secretory types. Inflammatory bowel disease includes ulcerative colitis and Crohn's disease. Parkinson's disease includes those occurring over age 50 and juvenile Parkinson's disease. Cancer includes lung cancer, breast cancer, stomach cancer, colon cancer, liver cancer, renal cancer, pancreatic cancer, uterine cancer, ovarian cancer, head and neck cancer, osteosarcoma, chondrosarcoma, rhabdomyosarcoma, leiomyosarcoma, fibrosarcoma, liposarcoma, hemangiosarcoma, leukemia, malignant lymphoma, and myeloma. Cognitive decline includes those due to aging and those due to dementia (e.g., Alzheimer's disease, cerebrovascular dementia, Lewy body dementia, juvenile dementia, and frontotemporal lobar degeneration). Atopic dermatitis is a disease showing chronic, itchy eczema that gets better and worse repeatedly, and it is known that the skin barrier function is impaired in atopic dermatitis. Anti-inflammatory uses include uses for suppression of inflammation in colitis, inhibition of NF-kappaB activation, and inhibition of IL-8 production.

Obesity is defined as excessive accumulation of fat in the body (BMI of 25 or higher). A disease with adverse health effects due to obesity or visceral adiposity is called obesity. Abnormal sugar metabolism means a condition in which sugar metabolism is abnormal. Abnormal sugar metabolism is specifically a condition in which the results of fasting blood glucose measurement, sugar tolerance test, casual blood glucose level, hemoglobin A1c, etc. are above the normal ranges, and includes diabetes mellitus and borderline diabetes mellitus. Diabetes mellitus is a disease in which high blood glucose levels persist chronically due to lack of insulin action, and includes type 1 diabetes mellitus and type 2 diabetes mellitus. Inflammatory bowel disease includes ulcerative colitis and Crohn's disease. Autism is a type of developmental disorder, and is a disorder in which specificity of interpersonal relationships and qualitative impairment of communication are observed. Because it is difficult to clearly delimit the boundaries of autism, and similar tendencies range from more mild conditions to normal individuals, it is sometimes called autism spectrum disorder. Atopic dermatitis is a disease showing chronic, itchy eczema that gets better and worse repeatedly, and it is known that the skin barrier function is impaired in atopic dermatitis.

Cancer includes lung cancer, breast cancer, stomach cancer, colon cancer, liver cancer, kidney cancer, pancreatic cancer, uterine cancer, ovarian cancer, head and neck cancer, osteosarcoma, chondrosarcoma, rhabdomyosarcoma, leiomyosarcoma, fibrosarcoma, liposarcoma, angiosarcoma, leukemia, malignant lymphoma, and myeloma. Inflammatory bowel disease includes ulcerative colitis and Crohn's disease.

Abnormal lipid metabolism, sometimes referred to as dyslipidemia or hyperlipidemia, is a condition in which there is too much LDL cholesterol or triglycerides or continuous too little HDL cholesterol in the blood. Abnormal lipid metabolism includes hyper-LDL-cholesterolemia (140 mg/dL or higher), borderline hyper-LDL-cholesterolemia (120 to 139 mg/dL), hypertriglyceridemia (hyper-neutral lipidemia, 150 mg/dL or higher), and hypo-HDL-cholesterolemia (lower than 40 mg/dL). Insulin resistance is a condition in which insulin is secreted into the blood by the pancreas, but the sensitivity of target organs to insulin is reduced and their actions are blunted. In the state of insulin resistance, the glucose uptake capacity of muscle and adipose tissue is reduced and gluconeogenesis cannot be suppressed in the liver, resulting in difficulty in lowering blood glucose levels and requiring more insulin to return blood glucose levels to normal. If this condition persists, the insulin secretion function of the pancreas declines and blood glucose levels rise, which is said to cause type II diabetes. Insulin resistance includes those caused by heredity, obesity, lack of exercise, high-fat diets, and stress. Food allergy is an immunologically mediated reaction that is caused by ingested food and causes such symptoms as urticaria, eczema, diarrhea, and coughing. Food allergy includes systemic ones as well as oral allergy syndrome, in which symptoms are often limited to the oral cavity. Inflammatory bowel disease includes ulcerative colitis and Crohn's disease.

Inflammatory bowel disease includes ulcerative colitis and Crohn's disease. Liver dysfunction (liver function abnormality) is a condition in which the liver is damaged for some reason. Usually, blood tests show abnormal values for AST, ALT, γGTP ALP, LDH, bilirubin, and other values relating to liver function. Liver dysfunction includes fatty liver (fatty liver disease), alcoholic fatty liver, nonalcoholic fatty liver disease (NAFLD, simple fatty liver), abnormal bile acid metabolism, hepatitis, and cirrhosis. Intestinal barrier dysfunction is defined as an injury to the intestinal barrier function for any cause. Intestinal epithelial cells form robust tight junctions that act as a physical barrier against foreign microorganisms and also protect the host through the formation of a thick mucin layer and secretion of antimicrobial peptides. Intestinal barrier function is thought to be impaired when there is an imbalance of intestinal bacteria. Intestinal barrier dysfunction is thought to be associated with chronic inflammation in Crohn's disease, ulcerative colitis, gastrointestinal disorders such as colorectal cancer, and metabolic diseases such as type 2 diabetes mellitus, nonalcoholic fatty liver, and cardiovascular disease. Metabolic syndrome is defined as a condition in which the waist circumference is 85 cm or larger for men and 90 cm or larger for women (for both men and women, equivalent to a visceral fat area of 100 cm² or larger on abdominal CT scan) and two or more out of three of blood pressure, blood glucose, and lipids are outside the standard values. The condition that two or more out of three of blood pressure, blood glucose, and lipids are out of the standard values is specifically when two or more of the following three conditions apply.
Blood pressure: highest (systolic) blood pressure of 130 mmHg or higher, lowest (diastolic) blood pressure of 85 mmHg or higher, or both
Blood glucose: fasting blood glucose of 110 mg/dL or higher
Lipids: triglycerides of 150 mg/dL or higher, HDL cholesterol lower than 40 mg/dL, or both

Developmental disorder is a condition characterized by behavioral and emotional characteristics from infancy due to differences in the way the brain works that are present from birth. Developmental disorder includes autism spectrum disorder (pervasive developmental disorder including autism and Asperger's syndrome), learning disability (limited learning disability), attention-deficit/hyperactivity disorder (ADHD), tic, and stuttering.

The composition can also be used for treating a disease or condition that can be ameliorated by controlling growth of bacteria of the genus *Faecalibacterium* in the intestinal tract. Such a disease or condition may be any of various diseases including irritable bowel syndrome, inflammatory bowel disease, Parkinson's disease, cancer, cognitive decline, atopic dermatitis, and others.

The composition can also be used for treating a disease or condition that can be ameliorated by controlling growth of bacteria of the genus *Akkermansia* in the intestinal tract. Such a disease or condition may be any of various diseases including obesity, abnormal sugar metabolism, inflammatory bowel disease, autism, atopic dermatitis, and others.

The composition can also be used for treating a disease or condition that can be ameliorated by controlling growth of bacteria of the genus *Blautia* in the intestinal tract. Such a disease or condition may be any of various diseases including obesity, abnormal sugar metabolism, diabetes mellitus, cancer, inflammatory bowel disease, and others.

The composition can also be used for treating a disease or condition that can be ameliorated by controlling growth of bacteria of the genus *Subdoligranulum* in the intestinal tract. Such a disease or condition may be any of various diseases including obesity, abnormal lipid metabolism, insulin resistance, diabetes mellitus, food allergy, inflammatory bowel disease, and others.

The composition can also be used for treating a disease or condition that can be ameliorated by controlling growth of bacteria of the genus *Bilophila* in the intestinal tract. Such a disease or condition may be any of various diseases including abnormal sugar metabolism, diabetes mellitus, obesity, cancer, appendicitis, inflammatory bowel disease, liver dysfunction, intestinal barrier dysfunction, metabolic syndrome, and others.

The composition can also be used for treating a disease or condition that can be ameliorated by controlling growth of bacteria of the genus *Sutterella* in the intestinal tract. Such a disease or condition may be any of various diseases including developmental disorder, metabolic syndrome, and others.

For the present invention, treating (treatment of) a disease or condition includes reducing the risk of developing the disease or condition, delaying or preventing the development of the disease or condition, and stopping or delaying the progression of the disease or condition. The therapy includes curative therapy (therapy to remove the cause) and symptomatic therapy (therapy to ameliorate symptoms). Actions for amelioration or treatment include medical actions performed by physicians and nurses, midwives, and others under the direction of physicians, and non-therapeutic actions performed by non-physicians, such as pharmacists, nutritionists (including registered dietitian nutritionists and sports nutritionists), public health nurses, midwives, nurses, clinical laboratory technicians, sports instructors, drug manufacturers, drug sellers, food manufacturers, food sellers, and others. Furthermore, prevention or reduction of the risk of developing disease or condition includes recommendations for intake of specific foods and nutritional guidance (including guidance on nutrition necessary for medical treatment of injured or sick persons and guidance on nutrition for the maintenance and promotion of good health).

### [Composition]

### (Food compositions, etc.)

The composition of the present invention can be in the form of a food composition or pharmaceutical composition. Food and pharmaceutical are not limited to those for humans, and may be those for animals other than human, unless especially indicated. The food may be a common food, functional food, or nutritional composition, or a therapeutic diet (diet for the purpose of therapy, for which a medical practitioner writes a dietary prescription, and which is cooked by a dietitian or the like according to the prescription), dietetic food, ingredient-modified food, care food, or therapy-supporting food, unless especially indicated. The food is not limited to a solid food, but it may be a food in the form of liquid, for example, drink, drinkable preparation, liquid food, or soup, unless especially indicated. Functional food refers to a food that can give a predetermined functionality to a living body, and includes health foods at large, such as foods for specified health uses (abbreviated as "Tokuho" in Japanese, including conditional foods for specified health use), foods with function claims, foods with health claims including foods with nutrient function claims, foods for special dietary uses, supplements (for example, those of various kinds of dosage forms such as tablet, coated tablet, sugar-coated tablet, capsule and solution), and cosmetic food (for example, diet foods). In the present invention, the "functional foods" include health foods to which the health claim based on the food standards of

### CODEX (JOINT FAO/WHO FOOD STANDARDS PROGRAMME CODEX ALIMENTARIUS COMMISSION) is applied.

### (Subject)

The composition of the present invention is suitable to be administered to subjects for whom it is desirable or necessary to control growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal tract; and subjects with a disease or condition that can be ameliorated by controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal tract. The subjects for whom it is desirable or necessary to control growth of any bacteria selected from the group consisting of bacteria of the genus *Faecalibacterium,* bacteria of the genus *Akkermansia,* bacteria of the genus *Blautia,* and bacteria of the genus *Subdoligranulum* in the intestinal tract include those with low numbers of any bacteria selected from the group consisting of bacteria of the genus *Faecalibacterium,* bacteria of the genus *Akkermansia,* bacteria of the genus *Blautia,* and bacteria of the genus *Subdoligranulum* in the intestinal tract. The subjects for whom it is desirable or necessary to control growth of any bacteria selected from the group consisting of bacteria of the genus *Bilophila,* and bacteria of the genus *Sutterella* in the intestinal tract include those with high numbers of any bacteria selected from the group consisting of bacteria of the genus *Bilophila* and bacteria of the genus *Sutterella* in the intestinal tract. For the present invention, the term administration is used in the sense of administering a pharmaceutical product to a subject as well as having a subject ingest a food or other substance other than a pharmaceutical product.

The age of the subject is not particularly limited, and the subject may be, for example, a neonate (within 28 days of birth); an infant (younger than 1 year of age); a young child (1 to 6 years of age); a child (7 years of age or older and younger than 15 years of age); an adult (15 years of age or older); or a person of 65 years of age or older.

### (Administration route etc.)

The composition of the present invention may be administered orally, parenterally, e.g., via a tube (gastrostomy or enterostomy), or intranasally, but oral administration is preferred.

The composition can be administered to the subject repeatedly or continuously over a long period of time. The period of time is not particularly limited, but in order for the effect to be fully recognized, the composition should be administered continuously over a relatively long period of time, e.g., over 3 days or longer, 1 week or longer, 2 weeks or longer, 1 month or longer, 3 months or longer, 6 months or longer, or 1 year or longer.

The composition may be administered routinely, in advance, such as when the risk is high, or when the need arises. The composition may be administered as a meal, before, after, or between meals, or when the disease or condition that is to be ameliorated by the composition occurs.

### (Dose and content)

The dose of the composition of the present invention may be such an amount that the desired effect is exerted. The dose can be appropriately set in consideration of various factors such as the age, weight, and symptoms of the subject.

The daily dose of the composition can be at least 0.1 mg, preferably at least 0.3 mg, more preferably at least 0.6 mg, even more preferably at least 1 mg, in terms of the amount of the active ingredient. The maximum amount of the active ingredient per day may be 10 g or less, 5 g or less, 1 g or less, 100 mg or less, 60 mg or less, 30 mg or less, or 15 mg or less, no matter how the minimum amount is defined. When two or more types of active ingredients are contained in the composition, the amount of active ingredient means the total amount of the active ingredients contained.

Administration may be once a day or multiple times a day, e.g., 2 to 10 times. The dose of the active ingredient per one time may be, for example, 0.01 mg or more, preferably 0.03 mg or more, more preferably 0.06 mg or more, further preferably 0.1 mg or more. The maximum amount of the active ingredient per one time may be 3 g or less, 1 g or less, 100 mg or less, 33 mg or less, 20 mg or less, 10 mg or less, or 5 mg or less, no matter how is the minimum amount is defined.

The content of the active ingredient in the composition may be appropriately determined according to the form of the composition. For example, the content of the active ingredient based on solid content of the composition can be 0.1% or more, preferably 0.3% or more, more preferably 0.6% or more, further preferably 1% or more. The maximum amount of the active ingredient based on solid content may be 50% or less, 30% or less, 20% or less, 10% or less, or 5% or less, no matter how the minimum amount is defined. For the present invention, % means mass percent unless otherwise stated.

When the composition is a liquid, the content of the active ingredient can be, for example, 0.01% or more, preferably 0.03% or more, more preferably 0.06% or more, further preferably 0.1% or more. The maximum content of the active ingredient when the composition is a liquid may be 5% or less, 3% or less, 2% or less, 1% or less, or 0.5% or less, no matter how the minimum content is defined.

### (Other ingredients and additives)

The composition of the present invention may contain other active ingredients or nutritional components that are acceptable as food or pharmaceutical. Examples of such ingredients include lipids (e.g., milk fat, vegetable fats, fats containing medium-chain fatty acids), proteins (e.g., milk proteins, milk protein concentrate (MPC), whey protein concentrate (WPC), whey protein isolate (WPI), α-lactalbumin (α-La), β-lactoglobulin (β-Lg), heat-denatured whey proteins and enzyme-treated whey proteins, amino acids (e.g., lysine, arginine, glycine, alanine, glutamic acid, leucine, isoleucine, and valine), carbohydrates other than human milk oligosaccharides and constituent sugars of human milk oligosaccharides (e.g., glucose, sucrose, fructose, maltose, trehalose, erythritol, maltitol, palatinose, xylitol, and dextrin), electrolytes (e.g., sodium, potassium, calcium, and magnesium salts), vitamins (e.g., vitamin A, vitamin B1, vitamin B2, vitamin B6, vitamin B 12, vitamin C, vitamin D, vitamin E, vitamin K, biotin, folic acid, pantothenic acid, and nicotinic acids), minerals (e.g., copper, zinc, iron, cobalt, and manganese), antibiotics, dietary fibers, and so forth.

The composition of the present invention may contain prebiotics other than human milk oligosaccharides. Prebiotics are indigestible food ingredients that selectively alter growth and activity of certain bacteria in the large intestine, thereby favorably affecting the host and improving the host's health. One or more types of prebiotics other than human milk oligosaccharides may be used in the composition.

Prebiotics other than human milk oligosaccharides are not particularly limited so long as they do not interfere with the effect of the active ingredient contained in the composition. Examples of prebiotics other than human milk oligosaccharides include galactooligosaccharides, fructooligosaccharides, xylooligosaccharides, isomaltooligosaccharides, raffinose, lactulose, lactosucrose, soy oligosaccharides, coffee oligosaccharides, dietary fibers, and gluconic acid.

The composition may also further contain an additive acceptable for foods or pharmaceuticals. Examples of such an additive include inactive carriers (solid and liquid carriers), excipients, surfactants, binders, disintegrating agents, lubricants, dissolving aids, suspending agents, coating agents, colorants, preservatives, buffering agents, pH adjustors, emulsifiers, stabilizers, sweeteners, antioxidants, perfumes, acidulants, and natural substances. More specific examples include water, other aqueous solvents, pharmaceutically acceptable organic solvents, collagen, polyvinyl alcohol, polyvinylpyrrolidone, carboxyvinyl polymer, sodium alginate, water-soluble dextran, water-soluble dextrin, carboxymethyl starch sodium, pectin, xanthan gum, gum arabic, casein, gelatin, agar, glycerin, propylene glycol, polyethylene glycol, vaseline, paraffin, stearyl alcohol, stearic acid, human serum albumin, mannitol, sorbitol, lactose, sucralose, stevia, aspartame, acesulfame potassium, citric acid, lactic acid, malic acid, tartaric acid, phosphoric acid, acetic acid, fruit juice, vegetable juice, and so forth.

### (Dosage form or shape)

The composition of the present invention in the form of a food may be prepared in an arbitrary form such as solid, liquid, mixture, suspension, powder, granule, paste, jelly, gel, and capsule. The composition of the present invention in the form of a food can be made in such an arbitrary form as dairy product, supplement, confectionery, drink, drinkable preparation, seasoning, processed food, daily dish, and soup. More specifically, the composition of the present invention may be in the form of liquid diet, semi-liquid diet, jelly, gel, powder, special formula powdered milk, special formula liquid milk, powdered and liquid milk for pregnant and nursing women, fermented milk, bar, mousse, chocolate, biscuit, ice cream, fermented milk, lactic acid bacteria beverage, lactic beverage, milk beverage, soft drink, tablet, cheese, bread, biscuit, cracker, pizza crust, food for sick persons, nutritional food, frozen food, processed food, or the like, or may be in a form of granule, powder, paste, thick solution or the like for being mixed in drink or food and then ingested. Granules and powders can be in the form of cubes or sticks (single-dose packets). For the present invention, special formula powdered milk means a product obtained from a product produced by processing a food made from raw milk, cow's milk, special cow's milk, or raw water buffalo's milk or a food made by using them as a main raw material, by adding nutrients necessary for infants and young children, and making the resulting composition into powder as defined in the "Ministerial Ordinance on Milk and Milk products Concerning Compositional Standards, etc. (hereinafter referred to as the "Ministerial Ordinance on Milk, etc.")". For the present invention, special formula liquid milk means a product obtained from a product produced by processing a food made from raw milk, cow's milk, special cow's milk, or raw water buffalo's milk or a food made by using them as a main raw material, by adding nutrients necessary for infants and young children, and making the resulting composition into liquid as defined in the Ministerial Ordinance on Milk, etc.

Special formula powdered milk and special formula liquid milk (these are sometimes collectively referred to as special formula powdered milk etc., and the explanation of the special formula powdered milk etc. may be made for special formula powdered milk as an example, but such an explanation shall also apply to special formula liquid milk) can be for infants, follow-up (as a supplement to weaning food), low birth weight infants, children, adults, expectant mothers, nursing mothers, elderly persons, those with allergies, those with lactose intolerance, and those with congenital metabolic disorders. Preferred examples of special formula powdered milk etc. are those for infants, follow-up, low birth weight infants, and children.

The composition of the present invention as a pharmaceutical can be in an arbitrary dosage form, for example, those suitable for oral administration including solid preparations such as tablet, granule, powder, pill, and capsule and liquid preparations such as solution, suspension, and syrup, gel, aerosol, or the like.

### (Others)

In the manufacture of the composition of the present invention, time of adding the active ingredient can be appropriately chosen. Unless the characteristics of the active ingredient are not markedly degraded, the time of the addition is not particularly limited. For example, the active ingredient can be blended by mixing it with raw materials. Alternatively, the active ingredient can be added at the final stage of the manufacture to produce a composition containing the active ingredient.

The composition of the invention can have an indication describing the purpose of use (intended use), or an indication describing that administration of the composition is recommended for specific subjects.

The composition of the present invention can have an indication describing that, with the composition or the active ingredient, growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal tract can be controlled, growth of any bacteria selected from the group consisting of bacteria of the genus *Faecalibacterium,* bacteria of the genus *Akkermansia,* bacteria of the genus *Blautia,* and bacteria of the genus *Subdoligranulum* in the intestinal tract can be promoted, growth of any bacteria selected from the group consisting of bacteria of the genus *Bilophila* and bacteria of the genus *Sutterella* in the intestinal tract can be suppressed, a disease or condition that can be ameliorated by controlling (preferably promoting) growth of any bacteria selected from the group consisting of bacteria of the genus *Faecalibacterium,* bacteria of the genus *Akkermansia,* bacteria of the genus *Blautia,* and bacteria of the genus *Subdoligranulum* can be treated, a disease or condition that can be ameliorated by controlling (preferably suppressing) growth of any bacteria selected from the group consisting of bacteria of the genus *Bilophila* and bacteria of the genus *Sutterella* in the intestinal tract can be treated, the composition can be used as a prebiotic or can be used as a synbiotic (a combination of a probiotic and a prebiotic), or administration of the composition is recommended for specific subjects. The indication may be a direct or indirect indication. Examples of the direct indication include descriptions on tangible articles such as the product itself, package, container, label, and tag, and examples of the indirect indication includes advertising and campaign activities using such places or means as web site, shop, pamphlet, exhibition, seminar such as media seminar, book, newspaper, magazine, television, radio, postal matter, E-mail, and sound.

Hereafter, the present invention will be more specifically explained with reference to examples. However, the technical scope of the present invention is not limited by these examples.

### Examples

### 1. Example concerning bacteria of the genus Faecalibacterium

### [Preparation of sugar solutions]

2'-FL (Jennewein Biotechnologie GmbH), 3'-SL (Carbosynth), 6'-SL (Tokyo Kasei Kogyo, #S0886), lactose (Fujifilm Wako Pure Chemicals Corporation, #128-00095), fucose (Fujifilm Wako Pure Chemicals Corporation, #068-05323), and Neu5Ac (Fujifilm Wako Pure Chemicals Corporation, #011-26173) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 80 µL was added to 8 mL of GAM semisolid medium without dextrose (Nissui, #05460) for glycolysis test and incubated at 37°C for 24 hours under anaerobic conditions. Each fecal culture was added to 7 wells in total of a 96-well deep well plate in a volume of 900 µL each. To the 7 wells, the sugar solutions (6 types) and sterilized water were added, respectively, in a volume of 100 µL each (final concentrations of the sugar solutions, 1 mass %). After incubation at 37°C for 48 hours under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/).

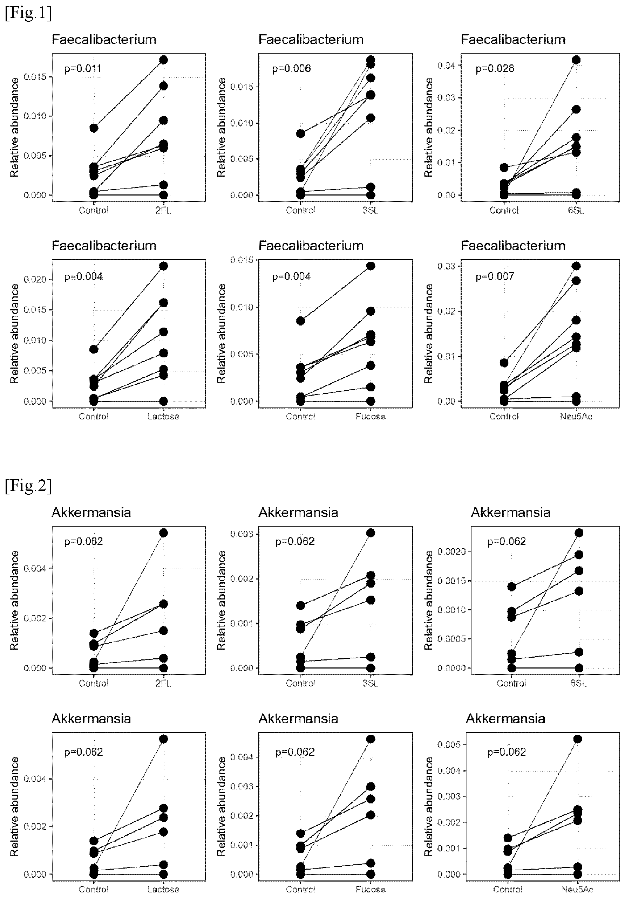

The results are shown in Fig. 1. Compared with the control group, the occupancy rate of bacteria of the genus *Faecalibacterium* significantly increased in the groups in which 2'-FL, 3'-SL, 6'-SL, lactose, fucose, and Neu5Ac were added. These results suggested that 2'-FL, 3'-SL, 6'-SL, lactose, fucose, and Neu5Ac promote growth of bacteria of the genus *Faecalibacterium* in the human intestinal tract. These results also suggested that both HMOs, fucosylated oligosaccharides and sialylated oligosaccharides, are effective. By the way, bacteria of the genus *Bifidobacterium* were detected in all the thawed fecal solutions.

In this example, a growth-promoting effect of lactose for bacteria of the genus *Faecalibacterium* was confirmed. HMOs contain lactose as a constituent sugar, and HMOs are thought to generate lactose in the human digestive tract after ingestion (Non-patent document 14). Therefore, it is considered that a growth-promoting effect for bacteria of the genus *Faecalibacterium* can be obtained with HMOs.

In this example, a growth-promoting effect of Neu5Ac for bacteria of the genus *Faecalibacterium* was confirmed. HMOs containing Neu5Ac as a constituent sugar are thought to generate Neu5Ac in the human digestive tract after ingestion (Non-patent documents 14 to 16). Therefore, it is considered that a growth-promoting effect for bacteria of the genus *Faecalibacterium* can be obtained with HMOs containing Neu5Ac as a constituent sugar (sialylated HMOs and HMOs containing sialic acid).

In this example, a growth-promoting effect of fucose for bacteria of the genus *Faecalibacterium* was confirmed. HMOs containing fucose as a constituent sugar are thought to generate fucose in the human digestive tract after ingestion (Non-patent documents 14 and 17). Therefore, it is considered that a growth-promoting effect for bacteria of the genus *Faecalibacterium* can be obtained with HMOs containing fucose as a constituent sugar (fucosylated HMOs and HMOs containing fucose).

In this example, a growth-promoting effect for bacteria of the genus *Faecalibacterium* was confirmed for all of 2'-FL, 3'-SL, and 6'-SL. From these results, it is considered that a growth-promoting effect for bacteria of the genus *Faecalibacterium* can be obtained with fucosylated HMOs and sialylated HMOs.

### 2. Example concerning bacteria of the genus Akkermansia

### [Preparation of sugar solutions]

2'-FL (Jennewein Biotechnologie GmbH), 3'-SL (Carbosynth), 6'-SL (Tokyo Kasei Kogyo, #S0886), lactose (Fujifilm Wako Pure Chemicals Corporation, #128-00095), fucose (Fujifilm Wako Pure Chemicals Corporation, #068-05323), and Neu5Ac (Fujifilm Wako Pure Chemicals Corporation, #011-26173) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 80 µL was added to 8 mL of GAM semisolid medium without dextrose (Nissui, #05460) for glycolysis test and incubated at 37°C for 24 hours under anaerobic conditions. Each fecal culture was added to 7 wells in total of a 96-well deep well plate in a volume of 900 µL each. To the 7 wells, the sugar solutions (6 types) and sterilized water were added, respectively, in a volume of 100 µL each (final concentrations of the sugar solutions, 1 mass %). After incubation at 37°C for 48 hours under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

The detection results for 6 subjects for whom bacteria of the genus *Akkermansia* were detected in feces are shown in Fig. 2. Compared with the sterile water group as the control, the occupancy rate of bacteria of the genus *Akkermansia* tended to increase in the groups in which 2'-FL, 3'-SL, 6'-SL, lactose, fucose, and Neu5Ac were added. These results suggested that 2'-FL, 3'-SL, 6'-SL, lactose, fucose, and Neu5Ac promote growth of bacteria of the genus *Akkermansia* in the human intestinal tract. Thus, these results suggested that both HMOs, fucosylated oligosaccharides and sialylated oligosaccharides, are effective. By the way, bacteria of the genus *Bifidobacterium* were detected in all the thawed fecal solutions.

In this example, a growth promotion of bacteria of the genus *Akkermansia* was confirmed with lactose. HMOs contain lactose as a constituent sugar, and HMOs are thought to generate lactose in the human digestive tract after ingestion (Non-patent document 14). Therefore, it is considered that a growth-promoting effect for bacteria of the genus *Akkermansia* can be obtained with HMOs.

In this example, a growth-promoting effect of Neu5Ac for bacteria of the genus *Akkermansia* was confirmed. HMOs containing Neu5Ac as a constituent sugar are thought to generate Neu5Ac in the human digestive tract after ingestion (Non-patent documents 14 to 16). Therefore, it is considered that a growth-promoting effect for bacteria of the genus *Akkermansia* can be obtained with HMOs containing Neu5Ac as a constituent sugar (sialylated HMOs and HMOs containing sialic acid).

In this example, a growth-promoting effect of fucose for bacteria of the genus *Akkermansia* was confirmed. HMOs containing fucose as a constituent sugar are thought to generate fucose in the human digestive tract after ingestion (Non-patent documents 14 and 17). Therefore, it is considered that a growth-promoting effect for bacteria of the genus *Akkermansia* can be obtained with HMOs containing fucose as a constituent sugar (fucosylated HMOs and HMOs containing fucose).

In this example, a growth-promoting effect for bacteria of the genus *Akkermansia* was confirmed for all of 2'-FL, 3'-SL, and 6'-SL. Therefore, it is considered that a growth-promoting effect for bacteria of the genus *Akkermansia* can be obtained with fucosylated HMOs and sialylated HMOs.

### 3. Example concerning bacteria of the genus Blautia

### [Preparation of sugar solutions]

2'-FL (Jennewein Biotechnologie GmbH), 3'-SL (Carbosynth), 6'-SL (Tokyo Kasei Kogyo, #S0886), lactose (Fujifilm Wako Pure Chemicals Corporation, #128-00095), fucose (Fujifilm Wako Pure Chemicals Corporation, #068-05323), and Neu5Ac (Fujifilm Wako Pure Chemicals Corporation, #011-26173) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 80 µL was added to 8 mL of GAM semisolid medium without dextrose (Nissui, #05460) for glycolysis test and incubated at 37°C for 24 hours under anaerobic conditions. Each fecal culture was added to 7 wells in total of a 96-well deep well plate in a volume of 900 µL each. To the 7 wells, the sugar solutions (6 types) and sterilized water were added, respectively, in a volume of 100 µL each (final concentrations of the sugar solutions, 1 mass %). After 48 hours of incubation at 37°C under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/).

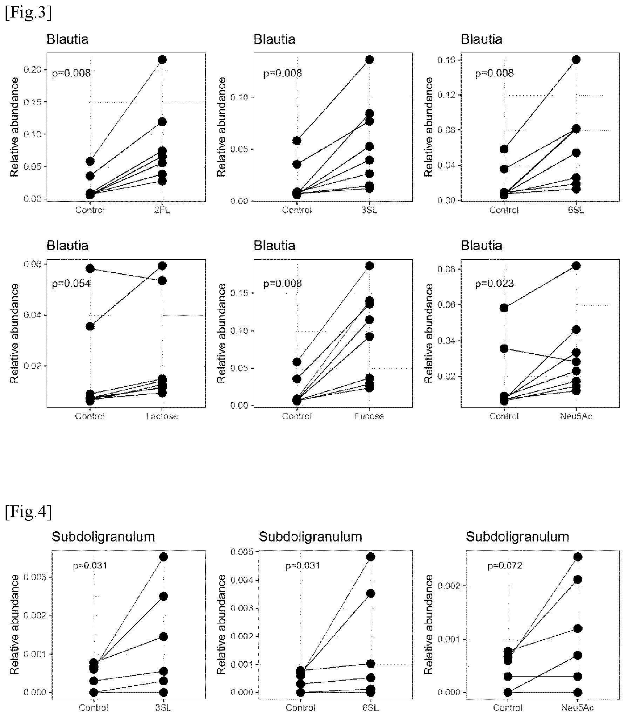

The results are shown in Fig. 3. Compared with the sterile water group as the control, the occupancy rate of bacteria of the genus *Blautia* significantly increased or tended to increase in the groups in which 2'-FL, 3'-SL, 6'-SL, lactose, fucose, and Neu5Ac were added. These results suggested that 2'-FL, 3'-SL, 6'-SL, lactose, fucose, and Neu5Ac promote growth of bacteria of the genus *Blautia* in the human intestinal tract. By the way, bacteria of the genus *Bifidobacterium* were detected in all the thawed fecal solutions.

In this example, a growth-promotion effect of lactose for bacteria of the genus *Blautia* was confirmed. HMOs contain lactose as a constituent sugar, and HMOs are thought to generate lactose in the human digestive tract after ingestion (Non-patent document 14). Therefore, it is considered that a growth-promoting effect for bacteria of the genus *Blautia* can be obtained with HMOs.

In this example, a growth-promoting effect of Neu5Ac for bacteria of the genus *Blautia* was confirmed. HMOs containing Neu5Ac as a constituent sugar are thought to generate Neu5Ac in the human digestive tract after ingestion (Non-patent documents 14 to 16). Therefore, it is considered that a growth-promoting effect for bacteria of the genus *Blautia* can be obtained with HMOs containing Neu5Ac as a constituent sugar (sialylated HMOs and HMOs containing sialic acid).

In this example, a growth-promoting effect of fucose for bacteria of the genus *Blautia* was confirmed. HMOs containing fucose as a constituent sugar are thought to generate fucose in the human digestive tract after ingestion (Non-patent documents 14 and 17). Therefore, it is considered that a growth-promoting effect for bacteria of the genus *Blautia* can be obtained with HMOs containing fucose as a constituent sugar (fucosylated HMOs and HMOs containing fucose).

In this example, a growth-promoting effect for bacteria of the genus *Blautia* was confirmed for all of 2'-FL, 3'-SL, and 6'-SL. Therefore, it is considered that a growth-promoting effect for bacteria of the genus *Blautia* can be obtained with fucosylated HMOs and sialylated HMOs.

### 4. Example concerning bacteria of the genus Subdoligranulum

### [Preparation of sugar solutions]

3'-SL (Carbosynth), 6'-SL (Tokyo Kasei Kogyo, #S0886), and Neu5Ac (Fujifilm Wako Pure Chemicals Corporation, #011-26173) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 60 µL was added to 6 mL of GAM semisolid medium without dextrose (Nissui, #05460) for glycolysis test and incubated at 37°C for 24 hours under anaerobic conditions. Each fecal culture was added to 4 wells in total of a 96-well deep well plate in a volume of 900 µL each. To the 4 wells, the sugar solutions (3'-SL, 6'-SL and Neu5Ac) and sterilized water were added, respectively, in a volume of 100 µL each (final concentrations of the sugar solutions, 1 mass %). After 48 hours of incubation at 37°C under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

The results are shown in Fig. 4. Compared with the sterile water group as the control, the occupancy rate of bacteria of the genus *Subdoligranulum* significantly increased or tended to increase in the groups in which 3'-SL, 6'-SL, and Neu5Ac were added. These results suggested that 3'-SL, 6'-SL, and Neu5Ac promote growth of bacteria of the genus *Subdoligranulum* in the human intestinal tract. By the way, bacteria of the genus *Bifidobacterium* were detected in all the thawed fecal solutions.

In this example, a growth-promoting effect of Neu5Ac for bacteria of the genus *Subdoligranulum* was confirmed. HMOs containing Neu5Ac as a constituent sugar are thought to generate Neu5Ac in the human digestive tract after ingestion (Non-patent documents 14 to 16). Therefore, it is considered that a growth-promoting effect for bacteria of the genus *Subdoligranulum* can be obtained with HMOs containing Neu5Ac as a constituent sugar (sialylated HMOs and HMOs containing sialic acid).

In this example, a growth-promoting effect for bacteria of the genus *Subdoligranulum* was confirmed for 3'-SL and 6'-SL. Therefore, it is considered that a growth-promoting effect for bacteria of the genus *Subdoligranulum* can be obtained with sialylated HMOs.

### 5. Example concerning bacteria of the genus Bilophila

### [Preparation of sugar solutions]

3'-SL (Carbosynth) and Neu5Ac (Fujifilm Wako Pure Chemicals Corporation, #011-26173) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 60 µL was added to 6 mL of GAM semisolid medium without dextrose (Nissui, #05460) for glycolysis test and incubated at 37°C for 24 hours under anaerobic conditions. Each fecal culture was added to 3 wells in total of a 96-well deep well plate in a volume of 900 µL each. To the 3 wells, the sugar solutions and sterilized water were added, respectively, in a volume of 100 µL each (final concentrations of the sugar solutions, 1 mass %). After incubation at 37°C for 24 hours under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

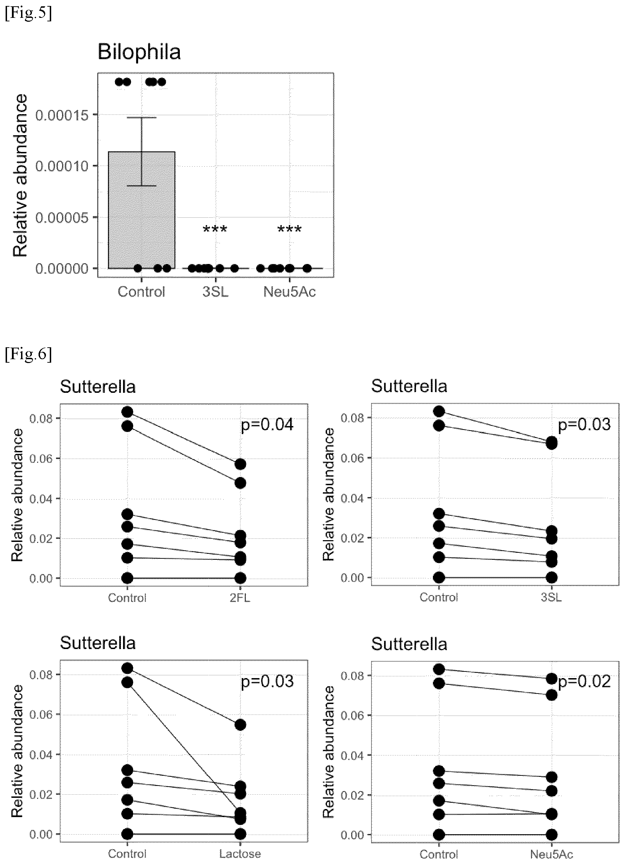

The results are shown in Fig. 5. Compared with the sterile water group as the control, the occupancy rate of bacteria of the genus *Bilophila* significantly decreased in the groups in which 3'-SL and Neu5Ac were added. These results suggested that 3'-SL and Neu5Ac suppress growth of bacteria of the genus *Bilophila* in the human intestinal tract. By the way, bacteria of the genus *Bifidobacterium* were detected in all the thawed fecal solutions.

In this example, a growth-suppressing effect of Neu5Ac for bacteria of the genus *Bilophila* was confirmed. HMOs containing Neu5Ac as a constituent sugar are thought to generate Neu5Ac in the human digestive tract after ingestion (Non-patent documents 14 to 16). Therefore, it is considered that a growth-suppressing effect for bacteria of the genus *Bilophila* can be obtained with HMOs containing Neu5Ac as a constituent sugar (sialylated HMOs and HMOs containing sialic acid).

In this example, a growth-suppressing effect for bacteria of the genus *Bilophila* was confirmed for 3'-SL. Therefore, it is considered that a growth-suppressing effect for bacteria of the genus *Bilophila* can be obtained with sialylated HMOs.

### 6. Example concerning bacteria of the genus Sutterella

### [Preparation of sugar solutions]

2'-FL (Jennewein Biotechnologie GmbH), 3'-SL (Carbosynth), lactose (Fujifilm Wako Pure Chemicals Corporation, #128-00095), and Neu5Ac (Fujifilm Wako Pure Chemicals Corporation, #011-26173) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 60 µL was added to 6 mL of GAM semisolid medium without dextrose (Nissui, #05460) for glycolysis test and incubated at 37°C for 24 hours under anaerobic conditions. Each fecal culture was added to 5 wells in total of a 96-well deep well plate in a volume of 900 µL each. To the 5 wells, the sugar solutions (4 types) and sterilized water were added, respectively, in a volume of 100 µL each (final concentrations of the sugar solutions, 1 mass %). After incubation at 37°C for 24 hours under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

The results are shown in Fig. 6. Compared with the sterile water group as the control, the occupancy rate of bacteria of the genus *Sutterella* significantly decreased in the groups in which 2'-FL, 3'-SL, lactose, and Neu5Ac were added. These results suggested that 2'-FL, 3'-SL, lactose, and Neu5Ac suppress growth of bacteria of the genus *Sutterella* in the human intestinal tract. By the way, bacteria of the genus *Bifidobacterium* were detected in all the thawed fecal solutions.

In this example, a growth-suppressing effect of lactose for bacteria of the genus *Sutterella* was confirmed. HMOs contain lactose as a constituent sugar, and HMOs are thought to generate lactose in the human digestive tract after ingestion (Non-patent document 14). Therefore, it is considered that a growth-suppressing effect for bacteria of the genus *Sutterella* can be obtained with HMOs.

In this example, a growth-suppressing effect of Neu5Ac for bacteria of the genus *Sutterella* was confirmed. HMOs containing Neu5Ac as a constituent sugar are thought to generate Neu5Ac in the human digestive tract after ingestion (Non-patent documents 14 to 16). Therefore, it is considered that a growth-suppressing effect for bacteria of the genus *Sutterella* can be obtained with HMOs containing Neu5Ac as a constituent sugar (sialylated HMOs and HMOs containing sialic acid).

In this example, a growth-suppressing effect for bacteria of the genus *Sutterella* was also confirmed for 2'-FL and 3'-SL. Therefore, it is considered that a growth-suppressing effect for bacteria of the genus *Sutterella* can be obtained with fucosylated HMOs and sialylated HMOs.

### [References cited in the examples]

Non-patent document 14: Masi AC, Stewart CJ. Untangling human milk oligosaccharides and infant gut microbiome. iScience. 2021;25(1):103542. Published 2021 Dec 1. doi:10.1016/j.isci.2021.103542
Non-patent document 15: Nishiyama K, Yamamoto Y, Sugiyama M, et al. Bifidobacterium bifidum Extracellular Sialidase Enhances Adhesion to the Mucosal Surface and Supports Carbohydrate Assimilation. mBio. 2017;8(5):e00928-17. Published 2017 Oct 3. doi:10.1128/mBio.00928-17
Non-patent document 16: Kiyohara M, Tanigawa K, Chaiwangsri T, Katayama T, Ashida H, Yamamoto K. An exo-alpha-sialidase from bifidobacteria involved in the degradation of sialyloligosaccharides in human milk and intestinal glycoconjugates. Glycobiology. 2011;21(4):437-447. doi:10.1093/glycob/cwq175
Non-patent document 17: Katayama T, Sakuma A, Kimura T, Makimura Y, Hiratake J, Sakata K, Yamanoi T, Kumagai H, Yamamoto K. Molecular cloning and characterization of Bifidobacterium bifidum 1,2-alpha-L-fucosidase (AfcA), a novel inverting glycosidase (glycoside hydrolase family 95). J Bacteriol. 2004 Aug;186(15):4885-93. doi: 10.1128/JB.186.15.4885-4893.2004. PMID: 15262925; PMCID: PMC451662

## Claims

1. A composition for controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, which contains any selected from the group consisting of a human milk oligosaccharide containing sialic acid as a constituent sugar and a constituent sugar thereof.

2. The composition according to claim 1, which contains any selected from the group consisting of N-acetylneuraminic acid, 3'-sialyllactose, and 6'-sialyllactose.

3. The composition according to claim 1, which contains any selected from the group consisting of N-acetylneuraminic acid and 3'-sialyllactose.

4. The composition according to claim 2, which is for controlling growth of any bacteria selected from the group consisting of bacteria of the genus *Faecalibacterium,* bacteria of the genus *Akkermansia,* bacteria of the genus *Blautia,* and bacteria of the genus *Subdoligranulum.*

5. The composition according to claim 1, which contains any selected from the group consisting of N-acetylneuraminic acid, 3'-sialyllactose, 6'-sialyllactose, fucose, and lactose, and is for controlling growth of any bacteria selected from the group consisting of bacteria of the genus *Faecalibacterium* and bacteria of the genus *Akkermansia.*

6. The composition according to claim 1, which contains lactose, and is for controlling growth of any bacteria selected from the group consisting of bacteria of the genus *Blautia* and bacteria of the genus *Sutterella.*

7. The composition according to any one of claims 1 to 6, which is for use as a prebiotic or synbiotic.

8. A composition for treating a disease or condition that can be ameliorated by controlling growth of any bacteria selected from the group consisting of bacteria of the genus *Faecalibacterium,* bacteria of the genus *Akkermansia,* bacteria of the genus *Blautia,* bacteria of the genus *Subdoligranulum,* bacteria of the genus *Bilophila,* and bacteria of the genus *Sutterella* in the intestinal bacterial microbiota, which contains any selected from the group consisting of a human milk oligosaccharide containing sialic acid as a constituent sugar and a constituent sugar thereof.

9. The composition according to claim 8, which contains any selected from the group consisting of N-acetylneuraminic acid, 3'-sialyllactose, and 6'-sialyllactose.

10. The composition according to any one of claims 1 to 6, which is for promoting growth of any bacteria selected from the group consisting of bacteria of the genus *Faecalibacterium,* bacteria of the genus *Akkermansia,* bacteria of the genus *Blautia,* and bacteria of the genus *Subdoligranulum,* or for suppressing growth of any bacteria selected from the group consisting of bacteria of the genus *Bilophila* and bacteria of the genus *Suterella.*

11. The composition according to claim 8 or 9, which is for treating a disease or condition that can be ameliorated by promoting growth of any bacteria selected from the group consisting of bacteria of the genus *Faecalibacterium,* bacteria of the genus *Akkermansia,* bacteria of the genus *Blautia,* and bacteria of the genus *Subdoligranulum,* or for treating a disease or condition that can be ameliorated by suppressing growth of any bacteria selected from the group consisting of bacteria of the genus *Bilophila* and bacteria of the genus *Sutterella.*
